(19) Europäisches Patentamt
European Patent Office
Office européen des brevets

(11) EP 0 964 856 B1

(12) **EUROPÄISCHE PATENTSCHRIFT**

(45) Veröffentlichungstag und Bekanntmachung des Hinweises auf die Patenterteilung:
**09.10.2002 Patentblatt 2002/41**

(21) Anmeldenummer: **98908063.5**

(22) Anmeldetag: **06.02.1998**

(51) Int Cl.[7]: **C07D 265/36**, C07D 319/20, C07D 413/12, C07D 405/12, A61K 31/535, A61K 31/335

(86) Internationale Anmeldenummer:
**PCT/EP98/00636**

(87) Internationale Veröffentlichungsnummer:
**WO 98/035949 (20.08.1998 Gazette 1998/33)**

(54) **BICYCLISCHE AROMATISCHE AMINOSÄUREN**

BICYCLIC AMINO ACIDS

AMINOACIDES AROMATIQUES BICYCLIQUES

(84) Benannte Vertragsstaaten:
**AT BE CH DE DK ES FI FR GB GR IE IT LI LU NL PT SE**
Benannte Erstreckungsstaaten:
**LT LV RO SI**

(30) Priorität: **13.02.1997 DE 19705450**

(43) Veröffentlichungstag der Anmeldung:
**22.12.1999 Patentblatt 1999/51**

(73) Patentinhaber: **MERCK PATENT GmbH**
**64271 Darmstadt (DE)**

(72) Erfinder:
- **DIEFENBACH, Beate**
  **D-64289 Darmstadt (DE)**
- **GOODMAN, Simon**
  **D-64287 Darmstadt (DE)**
- **MÄRZ, Joachim**
  **D-64521 Gross-Gerau (DE)**
- **RADDATZ, Peter**
  **D-64342 Seeheim (DE)**
- **RIPPMANN, Friedrich**
  **D-69120 Heidelberg (DE)**
- **WIESNER, Matthias**
  **D-55128 Mainz (DE)**

(56) Entgegenhaltungen:
**WO-A-94/12181**
**WO-A-95/32710**
**DE-A- 19 548 709**
**DE-A- 19 654 483**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 964 856 B1

Printed by Jouve, 75001 PARIS (FR)

## Beschreibung

Die Erfindung betrifft Verbindungen der Formel I

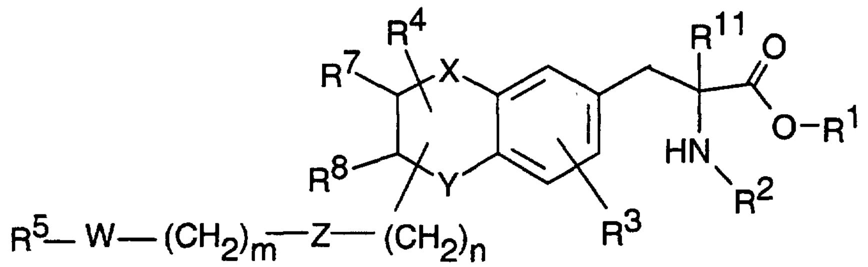

I

worin

| | |
|---|---|
| $R^1$ | H, Alkyl mit 1-6 C-Atomen oder Benzyl, |
| $R^2$ | $R^{10}$, CO-$R^{10}$, COOR$^6$, COOR$^{10}$, $SO_2R^6$ oder $SO_2R^{10}$, |
| $R^3$ | H, |
| $R^4$ | H oder =O, |
| $R^5$ | $NH_2$, $H_2N$-C(=NH) oder $H_2N$-(C=NH)-NH, wobei die primären Aminogruppen auch mit konventionellen Aminoschutzgruppen versehen sein können, oder ein-, zwei- oder dreifach durch $R^{10}$, CO-$R^{10}$, COOR$^{10}$ oder $SO_2R^{10}$ substituiert sein können, oder $R^6$, |
| $R^7$, $R^8$ | jeweils unabhängig voneinander fehlt oder H, |
| $R^7$ und $R^8$ | zusammen auch eine Bindung, |
| X | O oder N, |
| Y | O, |
| W, Z | jeweils unabhängig voneinander fehlt, O, S, NR$^1$, C(=O), CONH, NHCO, C(=S)NH, NHC(=S), C(=S), $SO_2NH$, $NHSO_2$ oder CA=CA', |
| $R^6$ | 1H-Imidazol-2-yl, Thiazol-2-yl, 1H-Benzimidazol-2-yl, 2H-Pyrazol-2-yl, 1 H-Tetrazol-5-yl, 2-Imino-imidazolidin-4-on-5-yl, 1-Alkyl-1,5-dihydro-imidazol-4-on-2-yl, Pyrimidin-2-yl oder 1,4,5,6-Tetrahydro-pyrimidin-2-yl, |
| $R^9$ | H, Hal, OA, NHA, NAA', NHAcyl, OAcyl, CN, $NO_2$, SA, SOA, $SO_2A$, $SO_2Ar$ oder $SO_3H$, |
| $R^{10}$ | H, A, Ar oder Aralkylen mit 7-14 C-Atomen, |
| $R^{11}$ | H oder Alkyl mit 1-6 C-Atomen, |
| A, A' | jeweils unabhängig voneinander H oder unsubstituiertes oder ein-, zwei- oder dreifach durch $R^9$ substituiertes Alkyl oder Cycloalkyl mit bis zu 15 C-Atomen und worin eine, zwei- oder drei Methylengruppen durch N, O und/oder S ersetzt sein können, |
| Ar | unsubstituiertes oder ein-, zwei- oder dreifach durch A und/oder $R^9$ substituiertes ein- oder zweikerniges aromatisches Ringsystem mit 0, 1, 2, 3 oder 4 N-, O- und/oder S-Atomen, |

| | |
|---|---|
| Acyl | Formyl, Acetyl, Propionyl, Butyryl, Trifluoracetyl oder Benzoyl, |
| Hal | F, Cl, Br oder I und |
| m, n | jeweils unabhängig voneinander 0, 1, 2, 3 oder 4 |

bedeuten,
sowie deren physiologisch unbedenklichen Salze.

Ähnliche Verbindungen sind z. B. aus WO 94/29273, WO 96/00730 und WO 96/18602 bekannt, ferner aus WO 9412181 und WO 9532710.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Vor allem wirken sie als Integrin-Inhibitoren, wobei sie insbesondere die Wechselwirkungen der $\alpha_v$-Integrin-Rezeptoren mit Liganden hemmen. Besondere Wirksamkeit zeigen die Verbindungen im Fall der Integrine $\alpha_V\beta_3$ und $\alpha_v\beta_5$. Ganz besonders wirksam sind die Verbindungen als Adhäsionsrezeptor-Antagonisten für den Vitronectin-Rezeptor $\alpha_v\beta_3$.
Diese Wirkung kann z.B. nach der Methode nachgewiesen werden, die von J.W. Smith et al. in J. Biol. Chem. 265, 11008-11013 und 12267-12271 (1990) beschrieben wird.
B. Felding-Habermann und D.A. Cheresh beschreiben in Curr. Opin. Cell. Biol. 5, 864 (1993) die Bedeutungen der Integrine als Adhäsionsrezeptoren für die unterschiedlichsten Phänomene und Krankheitsbilder, speziell in Bezug auf den Vitronectinrezeptor $\alpha_v\beta_3$.

Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science 264, 569-71 (1994) beschrieben.

Die Möglichkeit der Inhibierung dieser Wechselwirkung und damit zum Einleiten von Apoptose (programmierter Zelltod) angiogener vaskulärer Zellen durch ein cyclisches Peptid ist von P.C. Brooks, A.M. Montgomery, M. Rosenfeld, R.A. Reisfeld, T.-Hu, G. Klier und D.A. Cheresh in Cell 79, 1157-64 (1994) beschrieben.

Der experimentelle Nachweis, daß auch die erfindungsgemäßen Verbindungen die Anheftung von lebenden Zellen auf den entsprechenden Matrixproteinen verhindern und dementsprechend auch die Anheftung von Tumorzellen an Matrixproteine verhindern, kann in einem Zelladhäsionstest erbracht werden, der analog der Methode von F. Mitjans et al., J. Cell Science 108, 2825-2838 (1995) durchgeführt wird.

P.C. Brooks et al. beschreiben in J. Clin. Invest. 96, 1815-1822 (1995) $\alpha_v\beta_3$ -Antagonisten zur Krebsbekämpfung und zur Behandlung tumorinduzierter angiogener Krankheiten.
Die erfindungsgemäßen Verbindungen der Formel I können daher als Arzneimittelwirkstoffe insbesondere zur Behandlung von Tumorerkrankungen, Osteoporosen, osteolytischen Erkrankungen sowie zur Unterdrückung der Angiogenese eingesetzt werden.

Verbindungen der Formel I, die die Wechselwirkung von Integrinrezeptoren und Liganden, wie z. B. von Fibrinogen an den Fibrinogenrezeptor (Glycoprotein IIb/IIIa) blockieren, verhindern als GPIIb/IIIa-Antagonisten die Ausbreitung von Tumorzellen durch Metastase. Dies wird durch folgende Beobachtungen belegt:
Die Verbreitung von Tumorzellen von einem lokalen Tumor in das vaskuläre System erfolgt durch die Bildung von Mikroaggregaten (Mikrothromben) durch Wechselwirkung der Tumorzellen mit Blutplättchen. Die Tumorzellen sind durch den Schutz im Mikroaggregat abgeschirmt und werden von den Zellen des Immunsystems nicht erkannt.
Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPIIa/IIIb-Antagonisten als wirksame Metastase-Hemmer angesehen werden.

Verbindungen der Formel I hemmen neben der Bindung von Fibrinogen, Fibronectin und des Willebrand-Faktors an den Fibrinogenrezeptor der Blutplättchen auch die Bindung weiterer adhäsiver Proteine, wie Vitronectin, Kollagen und Laminin, an die entsprechenden Rezeptoren auf der Oberflache verschiedener Zelltypen. Sie verhindern insbesondere die Entstehung von Blutplättchenthromben und können daher zur Behandlung von Thrombosen, Apoplexie, Herzinfarkt, Entzündungen und Arteriosklerose eingesetzt werden.

Die Eigenschaften der Verbindungen können auch nach Methoden nachgewiesen werden, die in der EP-A1-0 462 960 beschrieben sind. Die Hemmung der Fibrinogenbindung an den Fibrinogenrezeptor kann nach der Methode nachgewiesen werden, die in der EP-A1-0 381 033 angegeben ist.

Die thrombozytenaggregationshemmende Wirkung läßt sich in vitro nach der Methode von Born (Nature 4832, 927-929, 1962) nachweisen.

Gegenstand der Erfindung sind demgemäß Verbindungen der Formel I nach Anspruch 1 und/oder ihrer physiologisch unbedenklichen Salze zur Herstellung eines Arzneimittels zur Verwendung als Integrin-Inhibitoren. Gegenstand der Erfindung sind insbesondere Verbindungen der Formel I nach Anspruch 1 und/oder ihrer unbedenklichen Salze, worin $R^2$ die Bedeutung Campher-10-sulfonyl hat, zur Herstellung eines Arzneimittels zur Bekämpfung von pathologisch angiogenen Erkrankungen, Tumoren, Osteoporose, Entzündungen und Infektionen.

Die Verbindungen der Formel I können als Arzneimittelwirkstoffe in der Human- und Veterinärmedizin eingesetzt werden, zur Prophylaxe und/oder Therapie von Thrombose myocardialem Infarkt, Arteriosklerose, Entzündungen, Apoplexie, Angina pectoris, Tumorerkrankungen, osteolytischen Krankheiten wie Osteoporose, pathologisch angiogenen Krankheiten wie z. B. Entzündungen, ophthalmologischen Krankheiten, diabetischer Retinopathie, makularer Degeneration, Myopia, okularer Histoplasmose, rheumatischer Arthritis, Osteoarthritis, rubeotischem Glaukom, ulcerativer Colitis, Morbus Crohn, Atherosklerose, Psoriasis, Restenose nach Angioplastie, viraler Infektion, bakterieller Infektion, Pilzinfektion, bei akutem Nierenversagen und bei der Wundheilung zur Unterstützung der Heilungsprozesse.

Die Verbindungen der Formel I können als antimikrobiell wirkende Substanzen bei Operationen eingesetzt werden, wo Biomaterialien, Implantate, Katheter oder Herzschrittmacher verwendet werden. Dabei wirken sie antiseptisch. Die Wirksamkeit der antimikrobiellen Aktivität kann durch das von P Valentin-Weigund et al., in Infection and Immunity, 2851-2855 (1988) beschriebene Verfahren nachgewiesen werden.

Gegenstand der Erfindung ist ferner ein Verfahren zur Herstellung von Verbindungen der Formel I nach Anspruch 1 sowie ihrer Salze, dadurch gekennzeichnet,

a) daß man eine Verbindung der Formel I aus einem ihrer funktionellen Derivate durch Behandeln mit einem solvolysierenden oder hydrogenolysierenden Mittel in Freiheit setzt,
oder

b) daß man eine Verbindung der Formel II

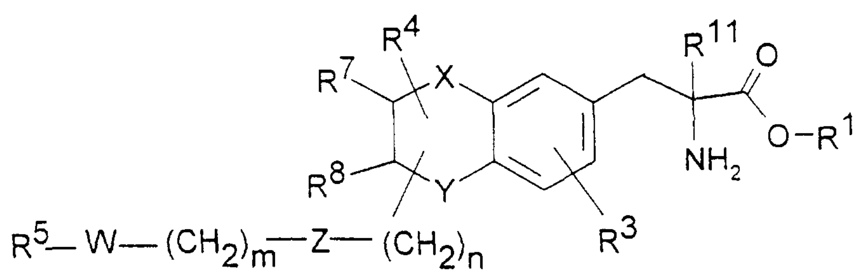

II

worin $R^1$, $R^3$, $R^4$, $R^5$, $R^7$, $R^8$ $R^{11}$, W, X, Y, Z, m und n die in Anspruch 1 angegebenen Bedeutungen haben, mit einer Verbindung der Formel III

$$R^2\text{-}L \qquad \text{III}$$

worin
$R^2$ die in Anspruch 1 angegebene Bedeutung hat
und L Cl, Br, I, OH oder eine reaktionsfähig veresterte OH-Gruppe bedeutet,
umsetzt,
oder

c) daß man einen Ester der Formel I verseift,
oder

d) daß man einen Rest $R^1$ und/oder $R^5$ in einen anderen Rest $R^1$ und/oder $R^5$ umwandelt,
und/oder

e) daß man eine basische oder saure Verbindung der Formel I durch Behandeln mit einer Säure oder Base in eines ihrere Salze überführt.

Die Verbindungen der Formel I besitzen mindestens ein chirales Zentrum und können daher in mehreren stereoisomeren Formen auftreten. Alle diese Formen (z. B. D- und L-Formen) und deren Gemische (z. B. die DL-Formen) sind in der Formel I eingeschlossen.
In die erfindungsgemäßen Verbindungen sind auch sogenannte Prodrug-Derivate eingeschlossen, d. h. mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Die vor- und nachstehend aufgeführten Abkürzungen stehen für:

| | |
|---|---|
| Ac | Acetyl |
| BOC | tert.-Butoxycarbonyl |
| CBZ oder Z | Benzyloxycarbonyl |
| DCCI | Dicyclohexylcarbodiimid |
| DMF | Dimethylformamid |
| DOPA | (3,4-Dihydroxyphenyl)-alanin |
| DPFN | 3,5-Dimethylpyrazol-1-formamidinium-nitrat |
| EDCI | N-Ethyl-N,N'-(dimethylaminopropyl)-carbodiimid |
| Et | Ethyl |
| Fmoc | 9-Fluorenylmethoxycarbonyl |
| HOBt | 1-Hydroxybenzotriazol |
| Me | Methyl |
| Mtr | 4-Methoxy-2,3,6-trimethylphenyl-sulfonyl |
| HONSu | N-Hydroxysuccinimid |
| OBn | Benzylester |
| OBut | tert.-Butylester |
| Oct | Octanoyl |
| OMe | Methylester |
| OEt | Ethylester |
| Orn | Ornithin |
| POA | Phenoxyacetyl |
| TBTU | O-(Benzotriazol-1-yl)-N,N,N,N-tetramethyluroniumtetrafluoroborat |
| TFA | Trifluoressigsäure |
| Trt | Trityl (Triphenylmethyl) |
| Z oder CBZ | Benzyloxycarbonyl. |

Für die gesamte Erfindung gilt, daß sämtliche Reste, die mehrfach auftreten, wie z.B. A und A', gleich oder verschieden sein können, d.h. unabhängig voneinander sind.
In den vorstehenden Formeln steht Alkyl vorzugsweise für Methyl, Ethyl, Propyl, Isopropyl, Butyl, Isobutyl, sek.-Butyl oder tert.-Butyl, ferner auch für Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1- , 1,2- , 1,3- , 2,2-, 2,3- oder 3,3-Dimethylbutyl, 1- oder 2-Ethylbutyl, 1-Ethyl-1-methylpropyl, 1-Ethyl-2-methylpropyl, 1,1,2-, 1,2,2-Trimethylpropyl, Heptyl, Octyl, Nonyl oder Decyl.
Cycloalkyl bedeutet vorzugsweise Cyclopropyl, Cyclobutyl, Cylopentyl, Cyclohexyl, Cycloheptyl oder 3-Menthyl. Cycloalkyl bedeutet insbesondere den Rest eines bicyclischen Terpens, ganz besonders bevorzugt ist der Campher-10-yl-Rest.
Alkylen bedeutet bevorzugt Methylen, Ethylen, Propylen, Butylen, Pentylen, ferner auch Hexylen, Heptylen, Ocytylen, Nonylen oder Decylen. Aralkylen bedeutet vorzugsweise Alkylenphenyl und ist z.B. vorzugsweise Benzyl oder Phenethyl.
Cycloalkylen bedeutet bevorzugt Cyclopropylen, 1,2- oder 1,3-Cyclobutylen, 1,2- oder 1,3-Cyclopentylen, 1,2- , 1,3- oder 1,4-Cyclohexylen, ferner 1,2-, 1,3- oder 1,4-Cycloheptylen.
CO-A ist Alkanoyl oder Cycloalkanoyl und bedeutet vorzugsweise Formyl, Acetyl, Propionyl, Butyryl, Pentanoyl, Hexanoyl, Heptanoyl, Octanoyl, Nonanoyl, Decanoyl, Undecanoyl, Dodecanoyl, Tridecanoyl, Tetradecanoyl, Pentadecanoyl, Hexadecanoyl, Heptadecanoyl oder Octadecanoyl.
Acyl bedeutet Formyl, Acetyl, Propionyl, Butyryl, Trifluoracetyl oder Benzoyl.
Bevorzugte Substituenten für Alkyl, Alkylen, Cycloalkyl, Cycloalkylen, Alkanoyl und Cycloalkanoyl sind z.B. Hal, OA, NHA, NAA', CN, $NO_2$, SA, SOA, $SO_2A$, $SO_2Ar$ und/oder $SO_3H$, insbesondere z.B. F, Cl, Hydroxy, Methoxy, Ethoxy, Amino, Dimethylamino, Methylthio, Methylsulfinyl, Methylsulfonyl oder Phenylsulfonyl.
Bevorzugte Substituenten für Ar und Arylen sind z.B. A und/oder Hal, OA, NHA, NAA', CN, $NO_2$, SA, SOA, $SO_2A$, $SO_2Ar$ und/oder $SO_3H$, insbesondere z.B. F, Cl, Hydroxy, Methoxy, Ethoxy, Amino, Dimethylamino, Methylthio, Methylsulfinyl, Methylsulfonyl oder Phenylsulfonyl.

In den Resten Alkyl, Alkylen, Cycloalkyl, Cycloalkylen, Alkanoyl und Cycloalkanoyl können jeweils eine, zwei- oder drei Methylengruppen durch N, O und/oder S ersetzt sein.

Ar-CO ist Aroyl und bedeutet vorzugsweise Benzoyl oder Naphthoyl

Ar ist unsubstituiertes, vorzugsweise - wie angegeben - monosubstituiertes Phenyl, im einzelnen bevorzugt Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Cyanphenyl, o-, m- oder p-Methoxyphenyl, o-, m- oder p-Ethoxyphenyl, o-, m- oder p-Fluorphenyl, o-, m- oder p-Bromphenyl, o-, m- oder p- Chlorphenyl, o-, m- oder p-Methylthiophenyl, o-, m- oder p-Methylsulfinylphenyl, o-, m- oder p-Methylsulfonylphenyl, o-, m- oder p-Aminophenyl, o-, m- oder p-Methylaminophenyl, o-, m- oder p-Dimethylaminophenyl, o-, m- oder p-Nitrophenyl,
weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Chlor-3-methyl-, 2-Chlor-4-methyl-, 2-Chlor-5-methyl-, 2-Chlor-6-methyl-, 2-Methyl-3-chlor-, 2-Methyl-4-chlor-, 2-Methyl-5-chlor-, 2-Methyl-6-chlor-, 3-Chlor-4-methyl-, 3-Chlor-5-methyl- oder 3-Methyl-4-chlorphenyl, 2-Brom-3-methyl-, 2-Brom-4-methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyl- oder 3-Methyl-4-bromphenyl, 2,4- oder 2,5-Dinitrophenyl, 2,5- oder 3,4-Dimethoxyphenyl, 2,3,4-, 2,3,5-, 2,3,6-, 2,4,6- oder 3,4,5-Trichlorphenyl, 2,4,6-Tritert.-Butylphenyl, 2,5-Dimethylphenyl, p-Iodphenyl, 4-Fluor-3-chlorphenyl, 4-Fluor-3,5-dimethylphenyl, 2-Fluor-4-bromphenyl, 2,5-Difluor-4-bromphenyl, 2,4-Dichlor-5-methylphenyl, 3-Brom-6-methoxyphenyl, 3-Chlor-6-methoxyphenyl, 2-Methoxy-5-methylphenyl, 2,4,6-Triisopropylphenyl, Naphthyl, 1,3-Benzodioxol-5-yl, 1,4-Benzodioxan-6-yl, Benzothiadiazol-5-yl oder Benzoxadiazol-5-yl.
Weiter bedeutet Ar vorzugsweise 2- oder 3-Furyl, 2- oder 3-Thienyl, 1-, 2-oder 3-Pyrrolyl, 1-, 2, 4- oder 5-Imidazolyl, 1-, 3-, 4- oder 5-Pyrazolyl, 2-, 4-oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4-H-Thiopyranyl, 3- oder 4-Pyridazinyl, Pyrazinyl, 2-, 3-, 4-, 5- 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7-oder 8-Cinnolinyl, 2-, 4-, 5-, 6-, 7- oder 8-Chinazolinyl.

Arylen hat die gleichen Bedeutungen wie für Ar angegeben, mit der Maßgabe, daß eine weitere Bindung vom aromatischen System zum nächsten Bindungsnachbar geknüpft ist.

Heterocycloalkylen bedeutet vorzugsweise 1,2-, 2,3- oder 1,3-Pyrrolidinyl, 1,2-, 2,4-, 4,5- oder 1,5-Imidazolidinyl, 1,2-, 2,3-, oder 1,3-Pyrazolidinyl, 2,3-, 3,4-, 4,5- oder 2,5-Oxazolidinyl, 1,2-, 2,3-, 3,4- oder 1,4- Isoxazolidinyl, 2,3-, 3,4-, 4,5- oder 2,5-Thiazolidinyl, 2,3-, 3,4-, 4,5- oder 2,5-Isothiazolidinyl, 1,2-, 2,3-, 3,4- oder 1,4-Piperidinyl, 1,4- oder 1,2-Piperazinyl, weiterhin bevorzugt 1,2,3-Tetrahydro-triazol-1,2- oder -1,4-yl, 1,2,4-Tetrahydro-triazol-1,2- oder 3,5-yl, 1,2- oder 2,5-Tetrahydrotetrazolyl, 1,2,3-Tetrahydro-oxadiazol-2,3-, -3,4-, -4,5- oder -1,5-yl, 1,2,4-Tetrahydro-oxadiazol-2,3-, -3,4- oder -4,5-yl, 1,3,4-Tetrahydro-thiadiazol-2,3-, -3,4-, -4,5- oder -1,5-yl, 1,2,4-Tetrahydro-thiadiazol-2,3-, -3,4-, -4,5-oder -1,5-yl, 1,2,3-Thiadiazol-2,3-, -3,4-, -4,5- oder -1,5-yl, 2,3- oder 3,4-Morpholinyl, 2,3-, 3,4- oder 2,4-Thiomorpholinyl.

$R^6$ bedeutet 1 H-Imidazol-2-yl, Thiazol-2-yl, 1H-Benzimidazol-2-yl, 2H-Pyrazol-2-yl, 1H-Tetrazol-5-yl, 2-Imino-imidazolidin-4-on-5-yl, 1-Alkyl-1,5-dihydro-imidazol-4-on-2-yl, Pyrimidin-2-yl oder 1,4,5,6-Tetrahydro-pyrimidin-2-yl.

$R^{11}$ bedeutet H oder Alkyl mit 1-6 C-Atomen, vorzugsweise H.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ig ausgedrückt werden, die der Formel I entsprechen und worin die nicht näher bezeichneten Reste die bei der Formel I angegebene Bedeutung haben, worin jedoch

in Ia)

| | |
|---|---|
| $R^1$ | H oder Alkyl mit 1-6 C-Atomen, |
| $R^2$ | $R^{10}$, CO-$R^{10}$, COO$R^{10}$ oder $SO_2R^{10}$, |
| $R^3$ | H, |
| $R^4$ | H oder =O, |
| $R^5$ | $H_2N$-C(=NH) oder $H_2N$-C(=NH)-NH, |
| W, Z | jeweils unabhängig voneinander fehlt, C(=O), NH, CONH oder NHCO, |
| X | O oder N, |
| Y | O, |

$R^{10}$ H, A oder Benzyl,
$R^{11}$ H,
A unsubstituiertes Alkyl oder Cycloalkyl mit bis zu 15 C-Atomen und
m, n jeweils unabhängig voneinander 0, 1 oder 2 bedeuten;

in Ib)

$R^1$ H oder Alkyl mit 1-6 C-Atomen,
$R^2$ $R^{10}$, CO-$R^{10}$, COOR$^{10}$ oder $SO_2R^{10}$,
$R^3$ H,
$R^4$ H oder =O,
$R^5$ $R^6$,
W, Z jeweils unabhängig voneinander fehlt, C(=O), NH, CONH oder NHCO,
X O oder N,
Y O,
$R^6$ 1 H-Imidazol-2-yl, Thiazol-2-yl, 1H-Benzimidazol-2-yl, 2H-Pyrazol-2-yl, 1H-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydro-pyrimidin-2-yl,
$R^{10}$ H, A oder Benzyl,
$R^{11}$ H,
A unsubstituiertes Alkyl oder Cycloalkyl mit bis zu 15 C-Atomen und
m, n jeweils unabhängig voneinander 0, 1 oder 2 bedeuten;

in Ic)

$R^1$ H oder Alkyl mit 1-6 C-Atomen,
$R^2$ $R^{10}$, CO-$R^{10}$, COOR$^{10}$ oder $SO_2R^{10}$,
$R^3$ H,
$R^4$ H oder =O,
$R^5$ $H_2N$-C(=NH) oder $H_2N$-C(=NH)-NH,
W, Z jeweils unabhängig voneinander fehlt, C(=O), NH, CONH oder NHCO,
X O oder N,
Y O,
A Alkyl mit 1-6 C-Atomen,
$R^{10}$ H, Alkyl mit 1-6 C-Atomen, Campher-10-yl oder Benzyl,
$R^{11}$ H,
m, n jeweils unabhängig voneinander 0, 1 oder 2 bedeuten;

in Id)

$R^1$ H oder Alkyl mit 1-6 C-Atomen,
$R^2$ $R^{10}$, CO-$R^{10}$, COOR$^{10}$ oder $SC_2R^{10}$,
$R^3$ H,
$R^4$ H oder =O,
$R^5$ $R^6$,
W, Z jeweils unabhängig voneinander fehlt, C(=O), NH, CONH oder NHCO,
X O oder N,
Y O,
$R^6$ 1 H-Imidazol-2-yl, Thiazol-2-yl, 1 H-Benzimidazol-2-yl, 2H-Pyrazol-2-yl, 1H-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydro-pyrimidin-2-yl,
$R^{10}$ H, Alkyl mit 1-4 C-Atomen, Campher-10-yl oder Benzyl,
$R^{11}$ H,
A unsubstituiertes Alkyl mit 1-6 C-Atomen und
m, n jeweils unabhängig voneinander 0, 1 oder 2 bedeuten;

in Ie)

| | |
|---|---|
| $R^1$ | H oder Alkyl mit 1-6 C-Atomen, |
| $R^2$ | $R^{10}$, CO-$R^{10}$, COOR$^{10}$ oder $SO_2R^{10}$, |
| $R^3$ | H, |
| $R^4$ | H oder =O, |
| $R^5$ | $R^6$, |
| W, Z | jeweils unabhängig voneinander fehlt, C(=O), NH, CONH oder NHCO, |
| X | O oder N, |
| Y | O, |
| $R^6$ | 1 H-Imidazol-2-yl, Thiazol-2-yl, 1H-Benzimidazol-2-yl, 2H-Pyrazol-2-yl, 1H-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydro-pyrimidin-2-yl, |
| $R^{10}$ | H, Alkyl mit 1-4 C-Atomen, Campher-10-yl oder Benzyl, |
| $R^{11}$ | H, |
| A | unsubstituiertes Alkyl mit 1-6 C-Atomen und |
| m, n | jeweils unabhängig voneinander 0, 1 oder 2 bedeuten; |

in If)

| | |
|---|---|
| $R^1$ | H oder Alkyl mit 1-6 C-Atomen, |
| $R^2$ | $R^{10}$, CO-$R^{10}$, COOR$^{10}$ oder $SO_2R^{10}$, |
| $R^3$ | H, |
| $R^4$ | H oder =O, |
| $R^5$ | $H_2N$-C(=NH) oder $H_2N$-C(=NH)-NH, |
| W, Z | jeweils unabhängig voneinander fehlt, C(=O), NH, CONH oder NHCO, |
| X | O oder N, |
| Y | O, |
| $R^{10}$ | Ar, |
| $R^{11}$ | H, |
| A | unsubstituiertes Alkyl oder Cycloalkyl mit bis zu 15 C-Atomen und |
| m, n | jeweils unabhängig voneinander 0, 1 oder 2 bedeuten; |

in Ig)

| | |
|---|---|
| $R^1$ | H oder Alkyl mit 1-6 C-Atomen, |
| $R^2$ | $R^{10}$, CO-$R^{10}$, COOR$^{10}$ oder $SO_2R^{10}$, |
| $R^3$ | H, |
| $R^4$ | H oder =O, |
| $R^5$ | $R^6$, |
| W, Z | jeweils unabhängig voneinander fehlt, C(=O), NH, CONH oder NHCO, |
| X | O oder N, |
| Y | O, |
| $R^6$ | 1 H-Imidazol-2-yl, Thiazol-2-yl, 1H-Benzimidazol-2-yl, 2H-Pyrazol-2-yl, 1H-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydro-pyrimidin-2-yl, |
| $R^{10}$ | Ar |
| $R^{11}$ | H, |
| A | unsubstituiertes Alkyl oder Cycloalkyl mit bis zu 15 C-Atomen und |
| m, n | jeweils unabhängig voneinander 0, 1 oder 2 bedeuten. |

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart;) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Verbindungen der Formel I können vorzugsweise erhalten werden, indem man Verbindungen der Formel I aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.

Es können auch mehrere - gleiche oder verschiedene - geschützte Aminound/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyloder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Die Abspaltung der Aminoschutzgruppe gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammoniumformiat (anstelle von Wasserstoff) an Pd/C in Methanol/DMF bei 20-30°.

Verbindungen der Formel I können vorzugsweise erhalten werden, indem man Verbindungen der Formel II mit Verbindungen der Formel III umsetzt. Die Ausgangsverbindungen der Formel II und III sind in der Regel neu. Sie können aber nach an sich bekannten Methoden hergestellt werden.

In den Verbindungen der Formel III bedeutet L vorzugsweise Cl, Br, I oder eine reaktionsfähig abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung der Verbindungen der Formel II erfolgt in der Regel in einem inerten Lösungsmittel, in Gegenwart eines säurebindenden Mittels vorzugsweise einer organischen Base wie Triethylamin, Dimethylanilin, Pyridin oder Chinolin. Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat, Wasser oder Gemische der genannten Lösungsmittel.

Weiterhin ist es möglich, einen Ester der Formel I zu verseifen. Zweckmäßig erfolgt dies durch Solvolyse oder Hydrogenolyse, wie oben angegeben, z.B. mit NaOH oder KOH in Dioxan-Wasser bei Temperaturen zwischen 0 und 60° C, vorzugsweise zwischen 10 und 40° C.

Ferner ist es möglich, daß man einen Rest $R^1$ und/oder $R^5$ in einen anderen Rest $R^1$ und/oder $R^5$ umwandelt. Insbesondere kann man eine Carbonsäure in einen Carbonsäureester umwandeln.
Die Umwandlung einer Cyangruppe in eine Amidinogruppe erfolgt durch Umsetzung mit z.B. Hydroxylamin und anschließender Reduktion des N-Hydroxyamidins mit Wasserstoff in Anwesenheit eines Katalysators wie z.B. Pd/C.
Ferner ist es möglich, eine konventionelle Aminoschutzgruppe durch Wasserstoff zu ersetzen, indem die Schutzgruppe, wie oben beschrieben, solvolytisch oder hydrogenolytisch abgespalten wird oder daß man eine durch eine konventionelle Schutzgruppe geschützte Aminogruppe durch Solvolyse oder Hydrogenolyse in Freiheit setzt.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits kann eine Säure der Formel I durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, ferner substituierte Ammoniumsalze, z. B. die Dimethyl-, Diethyloder Diisopropyl-ammoniumsalze, Monoethanol-, Diethanol- oder Diisopropylammoniumsalze, Cyclohexyl-, Dicyclohexylammoniumsalze, Dibenzylethylendiammoniumsalze, weiterhin z. B. Salze mit Arginin oder Lysin.

Die Verbindungen der Formel I enthalten ein oder mehrere chirale Zentren und können daher in racemischer oder in optisch-aktiver Form vorliegen.

Erhaltene Racemate können nach an sich bekannten Methoden mechanisch oder chemisch in die Enantiomeren getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoylphenylglycin) gefüllten Säule; als Laufmittel eignet sich z.B. ein Gemisch Hexan/Isopropanol/Acetonitril, z.B. im Volumenverhältnis 82:15:3.

Natürlich ist es auch möglich, optisch aktive Verbindungen der Formel I nach den oben beschriebenen Methoden zu erhalten, indem man Ausgangsstoffe verwendet, die bereits optisch aktiv sind.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale, topische Applikation oder für eine Applikation in Form eines Inhalation-Sprays eignen und mit den neuen

Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z. B. ein oder mehrere Vitamine.
Für die Applikation als Inhalationsspray können Sprays verwendet werden, die den Wirkstoff entweder gelöst oder suspendiert in einem Treibgas oder Treibgasgemisch (z. B. $CO_2$ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Die Verbindungen der Formel I und ihre physiologisch unbedenklichen Salze können als Integrininhibitoren bei der Bekämpfung von Krankheiten, insbesondere von pathologisch angiogenen Erkrankungen, Thrombosen, Herzinfarkt, koronaren Herzerkrankungen, Arteriosklerose, Tumoren, Entzündungen und Infektionen verwendet werden.
Bevorzugt sind Verbindungen der Formel I nach Anspruch 1 und/oder ihrer unbedenklichen Salze, worin $R^2$ die Bedeutung Campher-10-yl hat, zur Bekämpfung von pathologisch angiogenen Erkrankungen, Tumoren, Osteoporose, Entzündungen und Infektionen.

Dabei können die erfindungsgemäßen Substanzen in der Regel in Analogie zu anderen bekannten, im Handel befindlichen Peptiden, insbesondere aber in Analogie zu den in der US-A-4 472 305 beschriebenen Verbindungen verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

| | |
|---|---|
| Massenspektrometrie (MS): | EI (Elektronenstoß-Ionisation) $M^+$ |
| | FAB (Fast Atom Bombardment) $(M+H)^+$ |

Beispiel 1

Eine Lösung von 12 g BOC-3-nitro-L-tyrosin-benzylester ("1") in 200 ml THF wird bei Raumtemperatur und Normaldruck 6 Stunden in Gegenwart von 1 g Raney-Nickel hydriert. Man trennt den Katalysator ab und erhält nach üblicher Aufarbeitung 11,7 g BOC-3-amino-L-tyrosin-benzylester ("2"), FAB 387.
Eine Lösung von 9,3 g "2", 2,36 g Maleinsäureanhydrid und 3,3 ml Triethylamin in 150 ml DMF wird auf 80° erwärmt und 12 Stunden nachgerührt. Das Lösungsmittel wird entfernt und der Rückstand an Kieselgel mit Dichlormethan/Methanol 20:1 - 10:1 als Eluent chromatographiert. Man erhält 5,1 g (2S)-2-tert.-Butyloxycarboxamido-3-(3,4-dihydro-2-carboxymethyl-2H-1,4-benzoxazin-3-on-6-yl)-propionsäure-benzylester als Diastereomerengemisch ("3"), FAB 485.

Eine Lösung von 1 g "3" und 0,79 g 2-Chlor-1-methylpyridiniumiodid in 20 ml DMF wird mit 1 g Z-Guanidin und 1,75 ml Ethyldiisopropylamin versetzt und 12 Stunden bei Raumtemperatur gerührt. Man arbeitet wie üblich auf und erhält nach Chromatographie an Kieselgel (Toluol/Methanol 10:1) 0,2 g (2S)-2-tert.-Butyloxycarboxamido-3-[3,4-dihydro-2-(2-benzyloxycarbonylguanidino-2-oxoethyl)-2H-1,4-benzoxazin-3-on-6-yl]-propionsäurebenzylester ("4"), FAB 660.

Eine Lösung von 200 mg "4" und 3 ml Wasser/ 3 ml Dioxan wird in Gegenwart von 100 mg Palladium (10% auf Aktivkohle) bei RT und Normaldruck hydriert. Der pH-Wert wird durch Zugabe von 1N HCl bei 4 - 5 gehalten. Der Katalysator und die Lösungsmittel werden entfernt. Der Rückstand wird durch präparative HPLC gereinigt (RP-18 mit einem Gradienten Acetonitril/ Wasser + 0,3 % TFA von 1:80 zu 99:1 in einer Stunde) und man erhält 40 mg (2S)-2-tert.-Butyloxycarboxamido-3-[3,4-dihydro-2-(2-guanidino-2-oxoethyl)-2H-1,4-benzoxazin-3-on-6-yl]-propionsäure ("5"),

Trifluoracetat, FAB 660.

Beispiel 2

Eine Lösung von 6 g Z-L-DOPA-ethylester ("6") in 25 ml Ethanol und 25 ml Wasser wird unter Schutzgas mit 2,3 g Kaliumcarbonat versetzt. Man erwärmt auf 60°, fügt 4,5 ml Epibromhydrin zu und erwärmt auf 90°. Man rührt 2 Stunden nach, arbeitet wie üblich auf und reinigt das Rohprodukt an Kieselgel. Man erhält 5,6 g einer Mischung ("8") der stellungsisomeren Diastereomerenpaare, die sich nicht trennen lassen:

(2S)-2-Benzyloxycarboxamido-3-(3-(3R,3S)-hydroxymethyl-1,4-benzodioxan-6-yl)-propionsäure-ethylester ("7a") und
(2S)-2-Benzyloxycarboxamido-3-(2-(2R,2S)-hydroxymethyl-1,4-benzodioxan-6-yl)-propionsäure-ethylester ("7b"), FAB 416.

Eine Lösung von 2 g "8" in 30 ml Pyridin wird bei 0° mit 0,413 ml Methansulfonylchlorid versetzt und nach 2 Stunden Rühren wie üblich aufgearbeitet. Man erhält 2,2, g (2S)-2-Benzyloxycarboxamido-3-(2/3-methylsulfonyloxymethyl-1,4-benzodioxan-6-yl)-propionsäure-ethylester ("9"), FAB 494.

Eine Lösung von 1,6 g "9", 1,6 g Natriumazid und 30 ml DMF wird bei 75° 12 Stunden gerührt. Nach üblicher Aufarbeitung erhält man (2S)-2-Benzyloxycarboxamido-3-(2/3-azidomethyl-1,4-benzodioxan-6-yl)-propionsäureethylester ("10"), FAB 441.

Eine Lösung von 1,25 g "10" und 25 ml Methanol wird mit 3,4 ml 1N Natronlauge versetzt und 12 Stunden bei Raumtemperatur gerührt. Man arbeitet wie üblich auf und erhält 1,3 g (2S)-2-Benzyloxycarboxamido-3-(2/3-azidomethyl-1,4-benzodioxan-6-yl)-propionsäure ("11"), FAB 413.

In eine Lösung von 1,3 g "11" in 40 ml Pyridin und 20 ml Wasser leitet man bei Raumtemperatur 30 min Schwefelwasserstoff ein und läßt 12 Stunden stehen. Nach Entfernen der Lösungsmittel erhält man 1,5 g (2S)-2-Benzyloxycarboxamido-3-(2/3-aminomethyl-1,4-benzodioxan-6-yl)-propionsäure ("12"), FAB 387.

Eine Lösung von 0,3 g "12", 0,23 g DPFN und 0,22 ml Triethylamin in 10 ml DMF wird 12 Stunden bei 60° gerührt. Nach üblicher Aufarbeitung erhält man durch präparative HPLC (Bedingungen analog Beispiel 1 zur Reinigung von "5") eine Trennung der 2-Guanidinomethylverbindungen von den 3-Guanidinomethylverbindungen.
Ausbeute: 80 mg (2S)-2-Benzyloxycarboxamido-3-(2-(2R,S)-guanidinomethyl-1,4-benzodioxan-6-yl)-propionsäure ("13"), FAB 429.

Beispiel 3

Eine Lösung von 0,95 g BOC-Glycin und 0,96 g Carbonyldiimidazol in 20 ml THF wird 2 Stunden gerührt. Danach gibt man 0,7 g "12" dazu und rührt 12 Stunden nach. Nach üblicher Aufarbeitung erhält man 0,66 g (2S)-2-Benzyloxycarboxamido-3-(2/3-tert.-butyloxycarboxamido-acetamidomethyl-1,4-benzodioxan-6-yl)-propionsäure ("14"), FAB 544.

Eine Lösung von 0,15 g "14" in 5 ml Dichiormethan wird mit 0,5 ml Trifluoressigsäure versetzt und 8 Stunden gerührt. Nach Entfernen des Lösungsmittels werden 10 ml DMF zugesetzt und danach 80 mg DPFN und 70 µl Triethylamin hinzugegeben. Man erwärmt auf 80° und rührt 12 Stunden nach. Die Reinigung und Auftrennung der 2/3-lsomeren erfolgt durch präparative HPLC analog Beispiel 1.
Man erhält 42 mg (2S)-2-Benzyloxycarboxamido-3-(2-guanidino-acetamidomethyl-1,4-benzodioxan-6-yl)-propionsäure ("15"), FAB 486.

Beispiel 4

Durch eine Lösung von 0,45 g "14" in 10 ml Dioxan und 5 ml Wasser leitet man in Gegenwart von 0,2 g Palladium (10% auf Aktivkohle) 2 Stunden lang Wasserstoff. Nach Entfernen des Katalysators und üblicher Aufarbeitung erhält man 0,28 g (2S)-2-Amino-3-(2/3-tert.-butyloxycarboxamidoacetamidomethyl-1,4-benzodioxan-6-yl)-propionsäure ("16"), FAB 410.

Zu einer Lösung von 0,28 g "16" in 5 ml Acetonitril gibt man 430 µl N,O-Bis-(trimethylsilyl)-trifluoracetamid (BSTFA)und kocht anschließend 3 Stunden unter Rückfluß. Danach werden 66 µl Pyridin und 0,188 g R-Campher-10-sulfonsäurechlorid hinzugegeben und 3 Stunden bei 70° nachgerührt. Nach üblicher Aufarbeitung erhält man 0,26 g (2S)-2-(R)-Camphersulfonamido-3-(2/3-tert.-butyloxycarboxamido-acetamidomethyl-1,4-benzodioxan-6-yl)-propionsäure ("17"), FAB 624.

Analog der Herstellung von "15" erhält man nach Abspaltung der BOC-Gruppe und Guanylierung aus 0,25 g "17" 58 mg (2S)-2-(R)-Camphersulfonamido-3-(2-guanidino-acetamidomethyl-1,4-benzodioxan-6-yl)-propionsäure

("18"), FAB 566.

Analog erhält man durch Umsetzung von "16"
mit Butylsulfonylchlorid
(2S)-2-Butylsulfonamido-3-(2/3-tert.-butyloxycarboxamidoacetamidomethyl-1,4-benzodioxan-6-yl)-propionsäure;
mit 4-Tolylsulfonylchlorid
(2S)-2-(4-Tolylsulfonamido)-3-(2/3-tert.-butyloxycarboxamidoacetamidomethyl-1,4-benzodioxan-6-yl)-propionsäure;
mit Benzylsulfonylchlorid
(2S)-2-Benzylsulfonamido-3-(2/3-tert.-butyloxycarboxamidoacetamidomethyl-1,4-benzodioxan-6-yl)-propionsäure;
mit Phenylsulfonylchlorid
(2S)-2-Phenylsulfonamido-3-(2/3-tert.-butyloxycarboxamidoacetamidomethyl-1,4-benzodioxan-6-yl)-propionsäure;
mit 2-Naphthylsulfonylchlorid
(2S)-2-(2-Naphthylsulfonamido)-3-(2/3-tert.-butyloxycarboxamidoacetamidomethyl-1,4-benzodioxan-6-yl)-propionsäure
und mit Cylohexylsulfonylchlorid
(2S)-2-Cyclohexylsulfonamido-3-(2/3-tert.-butyloxycarboxamidoacetamidomethyl-1,4-benzodioxan-6-yl)-propionsäure.

Durch Abspaltung der BOC-Gruppe und Guanylierung erhält man daraus
(2S)-2-Butylsulfonamido-3-(2-guanidino-acetamidomethyl-1,4-benzodioxan-6-yl)-propionsäure;
(2S)-2-(4-Tolylsulfonamido)-3-(2-guanidino-acetamidomethyl-1,4-benzodioxan-6-yl)-propionsäure;
(2S)-2-Benzylsulfonamido-3-(2-guanidino-acetamidomethyl-1,4-benzodioxan-6-yl)-propionsäure;
(2S)-2-Phenylsulfonamido-3-(2-guanidino-acetamidomethyl-1,4-benzodioxan-6-yl)-propionsäure;
(2S)-2-(2-Naphthylsulfonamido)-3-(2-guanidino-acetamidomethyl-1,4-benzodioxan-6-yl)-propionsäure und
(2S)-2-Cyclohexylsulfonamido-3-(2-guanidino-acetamidomethyl-1,4-benzodioxan-6-yl)-propionsäure.

Beispiel 5

Eine Lösung von 0,3 g "3" und 0,248 g 2-Aminobenzimidazol ("A") in 10 ml DMF werden mit 0,26 g TBTU, 26 mg HOBT und 0,34 ml N-Methylmorpholin versetzt und 12 Stunden bei Raumtemperatur gerührt. Nach üblicher Aufarbeitung erhält man 0,14 g (2S)-2-tert.-Butyloxycarboxamido-3-{3,4-dihydro-2-[N-(2-benzimidazolyl)-carbamoylmethyl]-2H-1,4-benzoxazin-3-on-6-yl}-propionsäure-benzylester ("19").
Analog Beispiel 1 erhält durch Hydrierung aus "19" 60 mg (2S)-2-tert.-Butyloxycarboxamido-3-{3,4-dihydro-2-[N-(2-benzimidazolyl)-carbamoylmethyl]-2H-1,4-benzoxazin-3-on-6-yl}-propionsäure ("22"), FAB 510.

Analog erhält durch Umsetzung von "3"
mit 2-Aminoimidazol ("B")
(2S)-2-tert.-Butyloxycarboxamido-3-{3,4-dihydro-2-[N-(2-imidazolyl)-carbamoylmethyl]-2H-1,4-benzoxazin-3-on-6-yl}-propionsäure-benzylester ("20")
und anschließender Spaltung des Benzylesters
(2S)-2-tert.-Butyloxycarboxamido-3-{3,4-dihydro-2-[N-(2-imidazolyl)-carbamoylmethyl]-2H-1,4-benzoxazin-3-on-6-yl}-propionsäure ("23"), FAB 460;
und mit 2-Aminomethyl-benzimidazol ("C")
(2S)-2-tert.-Butyloxycarboxamido-3-{3,4-dihydro-2-[N-(2-benzimidazolylmethyl)-carbamoylmethyl]-2H-1,4-benzoxazin-3-on-6-yl}-propionsäure-benzylester ("21"),
und anschließender Spaltung des Benzylesters
(2S)-2-tert.-Butyloxycarboxamido-3-{3,4-dihydro-2-[N-(2-benzimidazolylmethyl)-carbamoylmethyl]-2H-1,4-benzoxazin-3-on-6-yl}-propionsäure ("24"), FAB 524.

Beispiel 6

Eine Lösung von 2 g "3" in 50 ml Dichlormethan wird mit 5 ml TFA versetzt und 1 Stunde bei Raumtemperatur gerührt. Nach Entfernen des Lösungsmittels erhält man 2 g (2S)-Amino-3-(3,4-dihydro-2-carboxymethyl-2H-1,4-benzoxazin-3-on-6-yl)-propionsäure-benzylester, Trifluoracetat ("25"), FAB 385.

Zu einer Lösung von 1 g "25" und 0,3 ml Triethylamin in 25 ml Acetonitril gibt man 1,2 ml BSTFA und kocht anschließend 2 Stunden unter Rückfluß gekocht. Bei 40° werden danach 0,19 ml Pyridin und 0,55 g (R)-Campher-10-sulfonsäurechlorid zugefügt und 12 Stunden bei 70° nachgerührt. Nach üblicher Aufarbeitung erhält man 0,41 g

(2S)-2-[(R)-Campher-sulfonamido]-3-(3,4-dihydro-2-carboxymethyl-2H-1,4-benzoxazin-3-on-6-yl)-propionsäure-benzylester ("26"), FAB 599.

Analog erhält man durch Umsetzung von "25"

mit Butylsulfonylchlorid

(2S)-2-Butylsulfonamido-3-(3,4-dihydro-2-carboxymethyl-2H-1,4-benzoxazin-3-on-6-yl)-propionsäure-benzylester;

mit 4-Tolylsulfonylchlorid

(2S)-2-(4-Tolylsulfonamido)-3-(3,4-dihydro-2-carboxymethyl-2H-1,4-benzoxazin-3-on-6-yl)-propionsäure-benzylester;

mit Benzylsulfonylchlorid

(2S)-2-Benzylsulfonamido-3-(3,4-dihydro-2-carboxymethyl-2H-1,4-benzoxazin-3-on-6-yl)-propionsäure-benzylester;

mit Phenylsulfonylchlorid

(2S)-2-Phenylsulfonamido-3-(3,4-dihydro-2-carboxymethyl-2H-1,4-benzoxazin-3-on-6-yl)-propionsäure-benzylester;

mit 2-Naphthylsulfonylchlorid

(2S)-2-(2-Naphthylsulfonamido)-3-(3,4-dihydro-2-carboxymethyl-2H-1,4-benzoxazin-3-on-6-yl)-propionsäure-benzylester

und mit Cylohexylsulfonylchlorid

(2S)-2-Cyclohexylsulfonamido-3-(3,4-dihydro-2-carboxymethyl-2H-1,4-benzoxazin-3-on-6-yl)-propionsäure-benzylester

Analog Beispiel 5 erhält man durch Umsetzung von "26"

mit "A"

(2S)-2-[(R)-Campher-sulfonamido]-3-{3,4-dihydro-2-[N-(2-benzimidazolyl)-carbamoylmethyl]-2H-1,4-benzoxazin-3-on-6-yl}-propionsäurebenzylester ("27"), FAB 714;

mit "B"

(2S)-2-[(R)-Campher-sulfonamido]-3-{3,4-dihydro-2-[N-(2-imidazolyl)-carbamoylmethyl]-2H-1,4-benzoxazin-3-on-6-yl}-propionsäure-benzylester

und mit "C"

(2S)-2-[(R)-Campher-sulfonamido]-3-{3,4-dihydro-2-[N-(2-benzimidazolylmethyl)-carbamoylmethyl)-2H-1,4-benzoxazin-3-on-6-yl}-propionsäure-benzylester ("29").

Durch Spaltung des Benzylesters mittels Hydrierung erhält man

aus "27"

(2S)-2-[(R)-Campher-sulfonamido]-3-{3,4-dihydro-2-[N-(2-benzimidazolyl)-carbamoylmethyl]-2H-1,4-benzoxazin-3-on-6-yl}-propionsäure ("28"), FAB 624

und aus "29"

(2S)-2-[(R)-Campher-sulfonamido]-3-{3,4-dihydro-2-[N-(2-benzimidazolylmethyl)-carbamoylmethyl]-2H-1,4-benzoxazin-3-on-6-yl}-propionsäure ("30"), FAB 638.

Analog erhält man durch Umsetzung

von (2S)-2-Butylsulfonamido-3-(3,4-dihydro-2-carboxymethyl-2H-1,4-benzoxazin-3-on-6-yl)-propionsäure-benzylester

mit "A"

(2S)-2-Butylsulfonamido-3-{3,4-dihydro-2-[N-(2-benzimidazolyl)-carbamoylmethyl]-2H-1,4-benzoxazin-3-on-6-yl}-propionsäure-benzylester,

mit "B"

(2S)-2-Butylsulfonamido-3-{3,4-dihydro-2-[N-(2-imidazolyl)-carbamoylmethyl]-2H-1,4-benzoxazin-3-on-6-yl}-propionsäure-benzylester,

mit "C"

(2S)-2-Butylsulfonamido-3-{3,4-dihydro-2-[N-(2-benzimidazolylmethyl)-carbamoylmethyl]-2H-1,4-benzoxazin-3-on-6-yl}-propionsäurebenzylester;

von (2S)-2-(4-Tolylsulfonamido)-3-(3,4-dihydro-2-carboxymethyl-2H-1,4-benzoxazin-3-on-6-yl)-propionsäure-benzylester

mit "A"

(2S)-2-(4-Tolylsulfonamido)-3-{3,4-dihydro-2-[N-(2-benzimidazolyl)-carbamoylmethyl]-2H-1,4-benzoxazin-3-on-6-yl}-propionsäure-benzylester,

mit "B"

(2S)-2-(4-Tolylsulfonamido)-3-{3,4-dihydro-2-[N-(2-imidazolyl)-carbamoylmethyl]-2H-1,4-benzoxazin-3-on-6-yl}-propionsäure-benzylester,

mit "C"

(2S)-2-(4-Tolylsulfonamido)-3-{3,4-dihydro-2-[N-(2-benzimidazolylmethyl)-carbamoylmethyl]-2H-1,4-benzoxazin-3-on-6-yl}-propionsäurebenzylester;

von (2S)-2-Benzylsulfonamido-3-(3,4-dihydro-2-carboxymethyl-2H-1,4-benzoxazin-3-on-6-yl)-propionsäure-benzylester

mit "A"

(2S)-2-Benzylsulfonamido-3-{3,4-dihydro-2-[N-(2-benzimidazolyl)-carbamoylmethyl]-2H-1,4-benzoxazin-3-on-6-yl}-propionsäure-benzylester,

mit "B"

(2S)-2-Benzylsulfonamido-3-{3,4-dihydro-2-[N-(2-imidazolyl)-carbamoylmethyl]-2H-1,4-benzoxazin-3-on-6-yl}-propionsäure-benzylester,

mit "C"

(2S)-2-Benzylsulfonamido-3-{3,4-dihydro-2-[N-(2-benzimidazolylmethyl)-carbamoylmethyl]-2H-1,4-benzoxazin-3-on-6-yl}-propionsäurebenzylester;

von (2S)-2-Phenylsulfonamido-3-(3,4-dihydro-2-carboxymethyl-2H-1,4-benzoxazin-3-on-6-yl)-propionsäure-benzylester

mit "A"

(2S)-2-Phenylsulfonamido-3-{3,4-dihydro-2-[N-(2-benzimidazolyl)-carbamoylmethyl]-2H-1,4-benzoxazin-3-on-6-yl}-propionsäure-benzylester,

mit "B"

(2S)-2-Phenylsulfonamido-3-{3,4-dihydro-2-[N-(2-imidazolyl)-carbamoylmethyl]-2H-1,4-benzoxazin-3-on-6-yl}-propionsäure-benzylester,

mit "C"

(2S)-2-Phenylsulfonamido-3-{3,4-dihydro-2-[N-(2-benzimidazolylmethyl)-carbamoylmethyl]-2H-1,4-benzoxazin-3-on-6-yl}-propionsäurebenzylester;

von (2S)-2-(2-Naphthylsulfonamido)-(3,4-dihydro-2-carboxymethyl-2H-1,4-benzoxazin-3-on-6-yl)-propionsäure-benzylester

mit "A"

(2S)-2-(2-Naphthylsulfonamido)-3-{3,4-dihydro-2-[N-(2-benzimidazolyl)-carbamoylmethyl]-2H-1,4-benzoxazin-3-on-6-yl}-propionsäurebenzylester,

mit "B"

(2S)-2-(2-Naphthylsulfonamido)-3-{3,4-dihydro-2-[N-(2-imidazolyl)-carbamoylmethyl]-2H-1,4-benzoxazin-3-on-6-yl}-propionsäure-benzylester,

mit "C"

(2S) -2-(2-Naphthylsulfonamido)-3-{3,4-dihydro-2-[N-(2-benzimidazolylmethyl)-carbamoylmethyl]-2H-1,4-benzoxazin-3-on-6-yl}propionsäure-benzylester;

von (2S)-2-Cyclohexylsulfonamido-3-(3,4-dihydro-2-carboxymethyl-2H-1,4-benzoxazin-3-on-6-yl)-propionsäure-benzylester

mit "A"

(2S)-2-Cyclohexylsulfonamido-3-{3,4-dihydro-2-[N-(2-benzimidazolyl)-carbamoylmethyl]-2H-1,4-benzoxazin-3-on-6-yl}-propionsäurebenzylester,

mit "B"

(2S)-2-Cyclohexylsulfonamido-3-{3,4-dihydro-2-[N-(2-imidazolyl)-carbamoylmethyl]-2H-1,4-benzoxazin-3-on-6-yl}-propionsäure-benzylester,

mit "C"

(2S) -2-Cyclohexylsulfonamido-3-{3,4-dihydro-2-[N-(2-benzimidazolylmethyl)-carbamoylmethyl]-2H-1,4-benzoxazin-3-on-6-yl}-propionsäurebenzylester.

Analog erhält man durch Spaltung der letztgenannten Benzylester mittels Hydrierung die nachstehenden Verbindungen

(2S)-2-Butylsulfonamido-3-{3,4-dihydro-2-[N-(2-benzimidazolyl)-carbamoylmethyl]-2H-1,4-benzoxazin-3-on-6-yl}-propionsäure;

(2S)-2-Butylsulfonamido-3-{3,4-dihydro-2-[N-(2-imidazolyl)-carbamoylmethyl]-2H-1,4-benzoxazin-3-on-6-yl}-propionsäure;

(2S)-2-Butylsulfonamido-3-{3,4-dihydro-2-[N-(2-benzimidazolylmethyl)-carbamoylmethyl]-2H-1,4-benzoxazin-3-on-6-yl}-propionsäure;

(2S)-2-(4-Tolylsulfonamido)-3-{3,4-dihydro-2-[N-(2-benzimidazolyl)-carbamoylmethyl]-2H-1,4-benzoxazin-3-on-6-yl}-propionsäure;

(2S)-2-(4-Tolylsulfonamido)-3-{3,4-dihydro-2-[N-(2-imidazolyl)-carbamoylmethyl]-2H-1,4-benzoxazin-3-on-6-yl}-propionsäure;
(2S)-2-(4-Tolylsulfonamido)-3-{3,4-dihydro-2-[N-(2-benzimidazolylmethyl)-carbamoylmethyl]-2H-1,4-benzoxazin-3-on-6-yl}-propionsäure;
(2S)-2-Benzylsulfonamido-3-{3,4-dihydro-2-[N-(2-benzimidazolyl)-carbamoylmethyl]-2H-1,4-benzoxazin-3-on-6-yl}-propionsäure;
(2S)-2-Benzylsulfonamido-3-{3,4-dihydro-2-[N-(2-imidazolyl)-carbamoylmethyl]-2H-1,4-benzoxazin-3-on-6-yl}-propionsäure;
(2S)-2-Benzylsulfonamido-3-{3,4-dihydro-2-[N-(2-benzimidazolylmethyl)-carbamoylmethyl]-2H-1,4-benzoxazin-3-on-6-yl}-propionsäure;
(2S)-2-Phenylsulfonamido-3-{3,4-dihydro-2-[N-(2-benzimidazolyl)-carbamoylmethyl]-2H-1,4-benzoxazin-3-on-6-yl}-propionsäure;
(2S)-2-Phenylsulfonamido-3-{3,4-dihydro-2-[N-(2-imidazolyl)-carbamoylmethyl]-2H-1,4-benzoxazin-3-on-6-yl}-propionsäure;
(2S)-2-Phenylsulfonamido-3-{3,4-dihydro-2-[N-(2-benzimidazolylmethyl)-carbamoylmethyl]-2H-1,4-benzoxazin-3-on-6-yl}-propionsäure;
(2S)-2-(2-Naphthylsulfonamido)-3-{3,4-dihydro-2-[N-(2-benzimidazolyl)-carbamoylmethyl]-2H-1,4-benzoxazin-3-on-6-yl}-propionsäure;
(2S)-2-(2-Naphthylsulfonamido)-3-{3,4-dihydro-2-[N-(2-imidazolyl)-carbamoylmethyl]-2H-1,4-benzoxazin-3-on-6-yl}-propionsäure;
(2S)-2-(2-Naphthylsulfonamido)-3-{3,4-dihydro-2-[N-(2-benzimidazolylmethyl)-carbamoylmethyl]-2H-1,4-benzoxazin-3-on-6-yl}propionsäure;
(2S)-2-Cyclohexylsulfonamido-3-{3,4-dihydro-2-[N-(2-benzimidazolyl)-carbamoylmethyl]-2H-1,4-benzoxazin-3-on-6-yl}-propionsäure;
(2S)-2-Cyclohexylsulfonamido-3-{3,4-dihydro-2-[N-(2-imidazolyl)-carbamoylmethyl]-2H-1,4-benzoxazin-3-on-6-yl}-propionsäure;
(2S)-2-Cyclohexylsulfonamido-3-{3,4-dihydro-2-[N-(2-benzimidazolylmethyl)-carbamoylmethyl]-2H-1,4-benzoxazin-3-on-6-yl}-propionsäure.

Beispiel 7

Durch Abspaltung der BOC-Gruppe mit TFA in Dichlormethan erhält man
aus "19"
(2S)-2-Amino-3-{3,4-dihydro-2-[N-(2-benzimidazolyl)-carbamoylmethyl]-2H-1,4-benzoxazin-3-on-6-yl}-propionsäure-benzylester ("31a"), FAB 500;
aus "20"
(2S)-2-Amino-3-{3,4-dihydro-2-[N-(2-imidazolyl)-carbamoylmethyl]-2H-1,4-benzoxazin-3-on-6-yl}-propionsäure-benzylester ("31b")
und aus "21"
(2S)-2-Amino-3-{3,4-dihydro-2-[N-(2-benzimidazolylmethyl)-carbamoylmethyl]-2H-1,4-benzoxazin-3-on-6-yl}-propionsäure-benzylester ("31c").

Eine Lösung von 0,13 g "31a" in 15 ml Dichlormethan wird mit 22 µl Butylsulfonsäurechlorid ("D") und 71 µl Triethylamin versetzt und 30 Stunden gerührt. Nach üblicher Aufarbeitung wird das Rohprodukt analog Beispiel 1 hydriert. Man erhält nach Reinigung durch präparative HPLC 13 mg (2S)-2-Butylsulfonamido-3-{3,4-dihydro-2-[N-(2-benzimidazolyl)-carbamoylmethyl]-2H-1,4-benzoxazin-3-on-6-yl}-propionsäure ("32a"), FAB 530;

Analog erhält man durch Umsetzung von "D" und anschließender Hydrierung
mit "31b"
(2S)-2-Butylsulfonamido-3-{3,4-dihydro-2-[N-(2-imidazolyl)-carbamoylmethyl]-2H-1,4-benzoxazin-3-on-6-yl}-propionsäure ("32b")
und mit "31c"
(2S)-2-Butylsulfonamido-3-{3,4-dihydro-2-[N-(2-benzimidazolylmethyl)-carbamoylmethyl]-2H-1,4-benzoxazin-3-on-6-yl}-propionsäure ("32c").

Beispiel 8

Eine Lösung von 2,1 g "1", 3,7 g 2,4-Dibromadipinsäurediethylester, 1,4 g Kaliumcarbonat und 0,137 g 18-Krone-6 in 100 ml Toluol wird 2 Stunden bei 80° gerührt. Nach üblicher Aufarbeitung erhält man 1,8 g (2S)-2-tert.-Butyloxycarboxamido-3-[3-nitro-4-(1,4-bis-(ethoxycarbonyl)-4-brombutyloxy)-phenyl]-propionsäure-benzylester ("34") als farblosen Sirup, FAB 696.

Eine Lösung von 1,5 g "34" und 0,7 g Natriumazid in 60 ml DMF wird bei 60° 12 Stunden gerührt. Nach üblicher Aufarbeitung erhält man 1,3 g (2S)-2-tert.-Butyloxycarboxamido-3-[3-nitro-4-(1,4-bis-(ethoxycarbonyl)-4-azidobutyloxy)-phenyl]-propionsäure-benzylester ("35"), FAB 658.

1,1 g "35" wird in 50 ml Methanol gelöst, mit 5,9 ml 1N NaOH versetzt und 5 Stunden gerührt. Nach üblicher Aufarbeitung erhält man 0,85 g (2S)-2-tert.-Butyloxycarboxamido-3-[3-nitro-4-(1,4-bis-carboxyl-4-azido-butyloxy)-phenyl]-propionsäure ("36"), FAB 512.

Eine Lösung von 0,5 g "36" in 10 ml Dioxan und 5 ml Wasser wird in Gegenwart von 0,1 g Palladium (10% auf Aktivkohle) 6 Stunden hydriert. Der pH-Wert wird mit 1 N HCl zwischen 4 und 6 gehalten. Nach Entfernen des Katalysators und der Lösungsmittel erhält man 0,21 g (2S)-2-tert.-Butyloxycarboxamido-3-[3,4-dihydro-2-(3-amino-3-carboxyl-propyl)-2H-1,4-benzoxazin-3-on-6-yl]-propionsäure ("37"), FAB 456.

Das Rohprodukt "37" (0,2 g) wird in 10 ml DMF, 2 ml Ethanol und 1 ml Wasser gelöst und mit 0,354 g DPFN in Gegenwart von 0,5 ml Triethylamin 24 Stunden bei 60° guanyliert. Nach üblicher Aufarbeitung erhält man 0,1 g (2S)-2-tert.-Butyloxycarboxamido-3-[3,4-dihydro-2-(3-guanidino-3-carboxyl-propyl)-2H-1,4-benzoxazin-3-on-6-yl]-propionsäure ("38"), FAB 480.

Eine Lösung von 50 mg "38" (Trifluoracetat) in 2 ml DMF wird mit 32 mg 2-Chlor-1-methylpyridiniumiodid und 60 µl Ethyldiisopropylamin vesetzt und 12 Stunden gerührt. Nach üblicher Aufarbeitung erhält man 22 mg (2S)-2-tert.-Butyloxycarboxamido-3-{3,4-dihydro-2-[3-(2-imino-4-oxo-imidazolidin-5-yl)-propyl]-2H-1,4-benzoxazin-3-on-6-yl}-propionsäure ("39").

<u>Beispiel 9</u>

Eine Lösung von (2S)-2-Butylsulfonamido-3-(3-hydroxymethyl-1,4-benzodioxan-6-yl)-propionsäure-benzylester in DMF wird mit äquimolaren Mengen Bromessigsäure-tert.-butylester und NaH versetzt. Man rührt 2 Stunden nach und erhält nach üblicher Aufarbeitung 2-Butylsulfonamido-3-(2-tert.-butoxycarbonylmethoxymethyl-2,3-dihydrobenzo[1,4]dioxin-6-yl)-propionsäure-benzylester.

Analog erhält man nach anschließender Abspaltung der BOC-Gruppe mit TFA, Umsetzung mit 2-Aminobenzimidazol und Spaltung des Benzylesters durch Hydrierung die Verbindung (2S)-2-Butylsulfonamido-3-{2-[(1Himidazol-2-ylcarbamoyl)-methoxymethyl]-2,3-dihydro-benzo[1,4]dioxin-6-yl}-propionsäure.

Analog erhält man 2-(4-Tolylsulfonamido)-3-{2-[(1H-imidazol-2-ylcarbamoyl)-methoxymethyl]-2,3-dihydrobenzo[1,4]dioxin-6-yl}propionsäure.

<u>Beispiel 10</u>

Durch Umsetzung von "25" mit Chlorameisensäure-2,2,2-trichlor-1,1-dimethyl-ethylester und anschließender Spaltung des Benzylesters durch Hydrierung erhält man die Verbindung (2S)-2-{[(2,2,2-Trichlor-1,1-dimethyl)-ethyl]-carboxamido}-3-(3,4-dihydro-2-carboxymethyl-2H-1,4-benzoxazin-3-on-6-yl)-propionsäure ("40").

Analog Beispiel 5 ergibt die Umsetzung von "40" mit "A" die Verbindung (2S)-2-{[(2,2,2-Trichlor-1,1-dimethyl)-ethyloxy]-carboxamido}-3-{3,4-dihydro-2-[N-(2-benzimidazoyl)-carbamoylmethyl]-2H-1,4-benzoxazin-3-on-6-yl}-propionsäure, Trifluoracetat, FAB 726

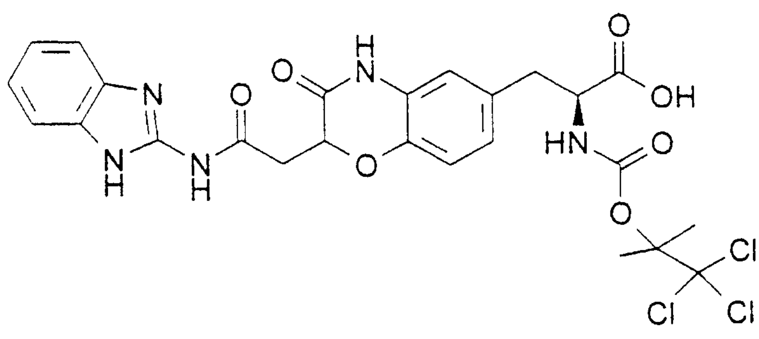

;

Analog erhält man die Verbindung (2S)-2-{[(Neopentyloxy)-ethyl]-carboxamido}-3-{3,4-dihydro-2-[N-(2-benzimidazoyl)-carbamoylmethyl]-2H-1,4-benzoxazin-3-on-6-yl}-propionsäure, FAB 524.

Beispiel 11

Durch Umsetzung von BOC-3-amino-L-tyrosin-ethylester mit (2S)-Brompentandisäure-5-benzylester [erhältlich durch Umsetzung von L-Glutaminsäure-γ-benzylester mit $NaNO_2$ und KBr in Schwefelsäure] und EDCI in Dichlormethan bei Raumtemperatur, erhält man nach 12 Stunden Rühren und üblicher Aufarbeitung die Verbindung (4S)-4-Brom-4-[5-((2S)-2-tert.butyloxycarbonylamino-2-ethoxycarbonylethyl)-2-hydroxy-phenylcarbamoyl]-buttersäurebenzylester, FAB 608.
Durch 12-stündiges Erhitzen mit DBU (Diazabicycloundec-7-en) in Toluol bei 100° erhält man nach üblicher Aufarbeitung die Verbindung (2S)-3-[(2R)-2-(2-Benzyloxycarbonyl-ethyl)-3-oxo-3,4-dihydro-2*H*-benzo[1,4]-oxazin-6-yl]-2-tert-butoxycarbonylamino-propionsäureethylester, FAB 527. Durch Hydrierung mit Pd/C erhält man (2S)-2-tert.-Butoxycarbonylamino-3-[(2R)-2-(2-carboxyethyl)-3-oxo-34-dihydro-2*H*-benzo[1,4]oxazin-6-yl]-propionsäureethylester ("41"), FAB 437

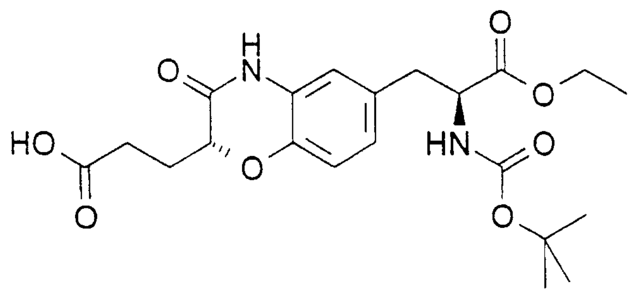

Analog Beispiel 5 erhält man durch Umsetzung von "41"
mit "A"
(2S)-2-tert.-Butyloxycarboxamido-3-{3,4-dihydro-2-[N-(2-benzimidazolyl)-carbamoylethyl]-(2R)-2H-1,4-benzoxazin-3-on-6-yl}propionsäure-ethylester ("42") und
mit "B"
(2S)-2-tert.-Butyloxycarboxamido-3-{3,4-dihydro-2-[N-(2-imidazolyl)-carbamoylethyl]-(2R)-2H-1,4-benzoxazin-3-on-6-yl}-propionsäureethylester ("43"), FAB 502.
Durch Spaltung des Ethylesters in "42" und "43" mit wässriger NaOH erhält man die Verbindungen
(2S)-2-tert.-Butyloxycarboxamido-3-{3,4-dihydro-2-[N-(2-benzimidazolyl)-carbamoylethyl]-(2R)-2H-1,4-benzoxazin-3-on-6-yl}propionsäure, FAB 524 und
(2S)-2-tert.-Butyloxycarboxamido-3-{3,4-dihydro-2-[N-(2-imidazolyl)-carbamoylethyl]-(2R)-2H-1,4-benzoxazin-3-on-6-yl}-propionsäure, FAB 474.
Analog erhält man die Verbindung
(2S)-2-tert.-Butyloxycarboxamido-3-{3,4-dihydro-2-[N-(2-imidazolyl)-carbamoylethyl]-(2S)-2H-1,4-benzoxazin-3-on-6-yl}-propionsäure, FAB 474.

Beispiel 12

Durch Abspaltung der BOC-Gruppe mit TFA in Dichlormethan erhält man aus "42" und "43" die nachstehenden Verbindungen
(2S)-2-Amino-3-{3,4-dihydro-2-[N-(2-benzimidazolyl)-carbamoylethyl]-(2R)-2H-1,4-benzoxazin-3-on-6-yl}-propionsäure-ethylester ("44") und
(2S)-2-Amino-3-{3,4-dihydro-2-[N-(2-imidazolyl)-carbamoylethyl]-(2R)-2H-1,4-benzoxazin-3-on-6-yl}-propionsäure-ethylester ("45"), FAB 402.
Analog Beispiel 6 erhält man durch Umsetzung von "44"
mit 2,3,5,6-Tetramethylphenylsulfonylchlorid
(2S)-2-(2,3,5,6-Tetramethylphenylsulfonamido)-3-{3,4-dihydro-2-[N-(2-benzimidazolyl)-carbamoylethyl]-(2R)-2H-1,4-benzoxazin-3-on-6-yl}-propionsäure-ethylester
und daraus durch Esterspaltung
(2S)-2-(2,3,5,6-Tetramethylphenylsulfonamido)-3-{3,4-dihydro-2-[N-(2-benzimidazolyl)-carbamoylethyl]-(2R)-2H-1,4-benzoxazin-3-on-6-yl}-propionsäure, FAB 620.
Analog erhält man durch Umsetzung von "45"
mit 3-Chlor-6-methoxy-phenylsulfonylchlorid

(2S)-2-(3-Chlor-6-methoxy-phenylsulfonamido)-3-{3,4-dihydro-2-[N-(2-imidazolyl)-carbamoylethyl]-(2R)-2H-1,4-benzoxazin-3-on-6-yl}-propionsäure-ethylester,

mit 1-Naphthylsulfonylchlorid

(2S)-2-(1-Naphthylsulfonamido)-3-{3,4-dihydro-2-[N-(2-imidazolyl)-carbamoylethyl]-(2R)-2H-1,4-benzoxazin-3-on-6-yl}-propionsaureethylester,

mit 2,3,5,6-Tetramethylphenylsulfonylchlorid

(2S)-2-(2,3,5,6-Tetramethylphenylsulfonamido)-3-{3,4-dihydro-2-[N-(2-imidazolyl)-carbamoylethyl]-(2R)-2H-1,4-benzoxazin-3-on-6-yl}-propionsäure-ethylester,

mit (R)-Campher-10-sulfonylchlorid

(2S)-2-[(R)-Campher-10-yl-sulfonamido)-3-{3,4-dihydro-2-[N-(2--imidazolyl)-carbamoylethyl]-(2R)-2H-1,4-benzoxazin-3-on-6-yl}-propionsäure-ethylester,

mit Butylsulfonylchlorid

(2S)-2-Butylsulfonamido-3-{3,4-dihydro-2-[N-(2-imidazolyl)-carbamoylethyl]-(2R)-2H-1,4-benzoxazin-3-on-6-yl}-propionsäureethylester,

mit Chlorameisensäureisopropylester

(2S)-2-Isopropoxycarboxamido-3-{3,4-dihydro-2-[N-(2-imidazolyl)-carbamoylethyl]-(2R)-2H-1,4-benzoxazin-3-on-6-yl}-propionsäureethylester,

mit Chlorameisensäureisobutylester

(2S)-2-Isobutoxycarboxamido-3-{3,4-dihydro-2-[N-(2-imidazolyl)-carbamoylethyl]-(2R)-2H-1,4-benzoxazin-3-on-6-yl}-propionsäureethylester,

mit Chlorameisensäureneopentylester

(2S)-2-Neopentyloxycarboxamido-3-{3,4-dihydro-2-[N-(2-imidazolyl)-carbamoylethyl]-(2R)-2H-1,4-benzoxazin-3-on-6-yl}-propionsäureethylester,

mit Chlorameisensäurebenzylester

(2S)-2-Benzyloxycarboxamido-3-{3,4-dihydro-2-[N-(2-imidazolyl)-carbamoylethyl]-(2R)-2H-1,4-benzoxazin-3-on-6-yl}-propionsäureethylester,

mit Benzylsulfonylchlorid

(2S)-2-Benzylsulfonamido-3-{3,4-dihydro-2-[N-(2-imidazolyl)-carbamoylethyl]-(2R)-2H-1,4-benzoxazin-3-on-6-yl}-propionsäureethylester,

mit Benzolsulfonylchlorid

(2S)-2-Benzolsulfonamido-3-{3,4-dihydro-2-[N-(2-imidazolyl)-carbamoylethyl]-(2R)-2H-1,4-benzoxazin-3-on-6-yl}-propionsäureethylester,

und daraus durch Esterspaltung die nachstehenden Propionsäurederivate

(2S)-2-(3-Chlor-6-methoxy-phenylsulfonamido)-3-{3,4-dihydro-2-[N-(2-imidazolyl)-carbamoylethyl]-(2R)-2H-1,4-benzoxazin-3-on-6-yl}-propionsäure, FAB 578;

(2S)-2-(1-Naphthylsulfonamido)-3-{3,4-dihydro-2-[N-(2-imidazolyl)-carbamoylethyl]-(2R)-2H-1,4-benzoxazin-3-on-6-yl}-propionsäure, FAB 564;

(2S)-2-(2,3,5,6-Tetramethylphenylsulfonamido)-3-{3,4-dihydro-2-[N-(2-imidazolyl)-carbamoylethyl]-(2R)-2H-1,4-benzoxazin-3-on-6-yl}-propionsäure, FAB 570;

(2S)-2-[(R)-Campher-10-yl-sulfonamido)-3-{3,4-dihydro-2-[N-(2--imidazolyl)-carbamoylethyl]-(2R)-2H-1,4-benzoxazin-3-on-6-yl}-propionsäure, FAB 588;

(2S)-2-Butylsulfonamido-3-{3,4-dihydro-2-[N-(2-imidazolyl)-carbamoylethyl]-(2R)-2H-1,4-benzoxazin-3-on-6-yl}-propionsäure, FAB 494;

(2S)-2-Isopropoxycarboxamido-3-{3,4-dihydro-2-[N-(2-imidazolyl)-carbamoylethyl]-(2R)-2H-1,4-benzoxazin-3-on-6-yl}-propionsäure, FAB 460;

(2S)-2-Isobutoxycarboxamido-3-{3,4-dihydro-2-[N-(2-imidazolyl)-carbamoylethyl]-(2R)-2H-1,4-benzoxazin-3-on-6-yl}-propionsäure, FAB 474;

(2S)-2-Neopentyloxycarboxamido-3-{3,4-dihydro-2-[N-(2-imidazolyl)-carbamoylethyl]-(2R)-2H-1,4-benzoxazin-3-on-6-yl}-propionsäure, FAB 488;

(2S)-2-Benzyloxycarboxamido-3-{3,4-dihydro-2-[N-(2-imidazolyl)-carbamoylethyl]-(2R)-2H-1,4-benzoxazin-3-on-6-yl}-propionsäure, FAB 508;

(2S)-2-Benzylsulfonamido-3-{3,4-dihydro-2-[N-(2-imidazolyl)-carbamoylethyl]-(2R)-2H-1,4-benzoxazin-3-on-6-yl}-propionsäure, FAB 528;

(2S)-2-Benzolsulfonamido-3-{3,4-dihydro-2-{N-(2-imidazolyl)-carbamoylethyl]-(2R)-2H-1,4-benzoxazin-3-on-6-yl}-propionsäure, FAB 514.

Analog erhält man die Verbindung (2S)-2-(1,1-Dimethyl-2,2,2-trichlorethyloxycarboxamido)-3-{3,4-dihydro-2-[N-(2-imidazolyl)-carbamoylethyl]-(2R)-2H-1,4-benzoxazin-3-on-6-yl}-propionsäure, FAB 578.

Beispiel 13

25 g L-Orn($N^{\delta}$-Z) werden mit 37 g Kaliumbromid in 300 ml 2,5n Schwefelsäure gelöst und bei 0° mit 9,7 g Natriumnitrit versetzt. Man läßt auf Raumtemperatur erwärmen und rührt 12 Stunden nach. Man arbeitet wie üblich auf und erhält 11 g (2S)-2-Brom-4-benzyloxycarbonylamino-buttersäure als Öl, EI 330.
Durch anschließende Umsetzung mit BOC-3-amino-L-tyrosin-ethylester und EDCI in Dichlormethan bei Raumtemperatur, erhält man nach 12 Stunden Rühren, üblicher Aufarbeitung und anschließender Umsetzung des Produktes mit DBU (Diazabicycloundec-7-en) in Toluol bei 100° die Verbindung (2S)-3-[(2R)-2-(3-Benzyloxycarbonylamino-propyl)-3-oxo-3,4-dihydro-2*H*-benzo[1,4]oxazin-6-yl]-2-tert.-butoxycarbonylamino-propionsäureethylester ("46"), FAB 556.

Durch Esterhydrolyse mit wässriger Natronlauge und anschließender Abspaltung der Z-Gruppe durch Hydrierung (Pd/C) in Dioxan/Wasser erhält man die Verbindung (2S)-3-[(2R)-2-(3-Aminopropyl)-3-oxo-3,4-dihydro-2*H*-benzo[1,4]oxazin-6-yl]-2-tert.-butoxycarbonylamino-propionsäure, FAB 394.
Analog Beispiel 3 erhält man daraus durch Umsetzung mit DPFN die Verbindung (2S)-3-[(2R)-2-(3-Guanidinopropyl)-3-oxo-3,4-dihydro-2*H*benzo[1,4]oxazin-6-yl]-2-tert.-butoxycarbonylamino-propionsäure, FAB 436.

Durch Abspaltung der BOC-Gruppe mit TFA in Dichlormethan erhält man aus "46" die Verbindung (2S)-2-Amino-3-[(2R)-2-(3-benzyloxycarbonylamino-propyl)-3-oxo-3,4-dihydro-2*H*-benzo[1,4]oxazin-6-yl]-propionsäureethylester ("47"), Trifluoracetat, FAB 456

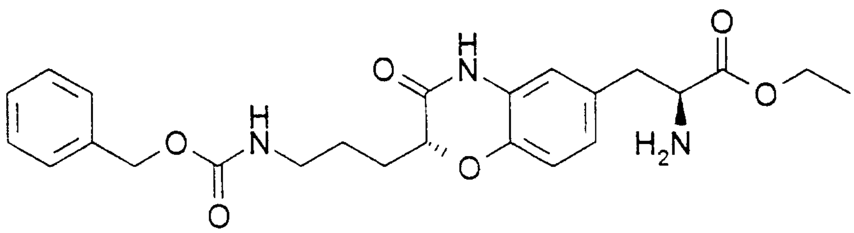

Analog Beispiel 6 erhält man durch Umsetzung von "47"
mit 2,3,5,6-Tetramethylphenylsulfonylchlorid
(2S)-2-(2,3,5,6-Tetramethylphenylsulfonamido)-3-{3,4-dihydro-2-(3-benzyloxycarbonylamino-propyl)-(2R)-2H-1,4-benzoxazin-3-on-6-yl}propionsäure-ethylester,
mit 3-Chlor-6-methoxy-phenylsulfonylchlorid
(2S)-2-(3-Chlor-6-methoxy-phenylsulfonamido)-3-{3,4-dihydro-2-(3-benzyloxycarbonylamino-propyl)-(2R)-2H-1,4-benzoxazin-3-on-6-yl}propionsäure-ethylester,
mit 1-Naphthylsulfonylchlorid
(2S)-2-(1-Naphthylsulfonamido)-3-{3,4-dihydro-2-(3-benzyloxycarbonylamino-propyl)-(2R)-2H-1,4-benzoxazin-3-on-6-yl}-propionsäureethylester,
mit 2,3,5,6-Tetramethylphenylsulfonylchlorid
(2S)-2-(2,3,5,6-Tetramethylphenylsulfonamido)-3-{3,4-dihydro-2-(3-benzyloxycarbonylamino-propyl)-(2R)-2H-1,4-benzoxazin-3-on-6-yl}propionsäure-ethylester,
mit (R)-Campher-10-sulfonylchlorid
(2S)-2-[(R)-Campher-10-yl-sulfonamido)-3-{3,4-dihydro-2-(3-benzyloxycarbonylamino-propyl)-(2R)-2H-1,4-benzoxazin-3-on-6-yl}propionsäure-ethylester,
mit Butylsulfonylchlorid
(2S)-2-Butylsulfonamido-3-{3,4-dihydro-2-(3-benzyloxycarbonylamino-propyl)-(2R)-2H-1,4-benzoxazin-3-on-6-yl}-propionsäure-ethylester, FAB 576;
mit Chlorameisensäureisopropylester
(2S)-2-Isopropoxycarboxamido-3-{3,4-dihydro-2-(3-benzyloxycarbonylamino-propyl)-(2R)-2H-1,4-benzoxazin-3-on-6-yl}-propionsäureethylester,
mit Chlorameisensäureisobutylester
(2S)-2-Isobutoxycarboxamido-3-{3,4-dihydro-2-(3-benzyloxycarbonylamino-propyl)-(2R)-2H-1,4-benzoxazin-3-on-6-yl}-propionsäure-ethylester,
mit Chlorameisensäureneopentylester
(2S)-2-Neopentyloxycarboxamido-3-{3,4-dihydro-2-(3-benzyloxycarbonylamino-propyl)-(2R)-2H-1,4-benzoxazin-3-on-6-yl}-propionsäureethylester, FAB 570;
mit Chlorameisensäurebenzylester
(2S)-2-Benzyloxycarboxamido-3-{3,4-dihydro-2-(3-benzyloxycarbonylamino-propyl)-(2R)-2H-1,4-benzoxazin-3-on-6-yl}-propionsäure-ethylester,
mit Benzylsulfonylchlorid
(2S)-2-Benzylsulfonamido-3-{3,4-dihydro-2-(3-benzyloxycarbonylamino-propyl)-(2R)-2H-1,4-benzoxazin-3-on-6-yl}-propionsäure-ethylester,
mit Benzolsulfonylchlorid
(2S)-2-Benzolsulfonamido-3-{3,4-dihydro-2-(3-benzyloxycarbonylamino-propyl)-(2R)-2H-1,4-benzoxazin-3-on-6-yl}-propionsäure-ethylester.

Aus den letztgenannten Z-geschützten Propionsäureestern erhält man durch Esterspaltung und Hydrierung die nachstehenden Verbindungen
(2S)-2-(2,3,5,6-Tetramethylphenylsulfonamido)-3-{3,4-dihydro-2-(3-amino-propyl)-(2R)-2H-1,4-benzoxazin-3-on-6-yl}-propionsäure,
(2S)-2-(3-Chlor-6-methoxy-phenylsulfonamido)-3-{3,4-dihydro-2-(3-amino-propyl)-(2R)-2H-1,4-benzoxazin-3-on-6-yl}-propionsäure,
(2S)-2-(1-Naphthylsulfonamido)-3-{3,4-dihydro-2-(3-amino-propyl)-(2R)-2H-1,4-benzoxazin-3-on-6-yl}-propionsäure,
(2S)-2-(2,3,5,6-Tetramethylphenylsulfonamido)-3-{3,4-dihydro-2-(3-amino-propyl)-(2R)-2H-1,4-benzoxazin-3-on-6-yl}-propionsäure,
(2S)-2-[(R)-Campher-10-yl-sulfonamido)-3-{3,4-dihydro-2-(3-aminopropyl)-(2R)-2H-1,4-benzoxazin-3-on-6-yl}-propionsäure,
(2S)-2-Butylsulfonamido-3-{3,4-dihydro-2-(3-amino-propyl)-(2R)-2H-1,4-benzoxazin-3-on-6-yl}-propionsäure,
(2S)-2-Isopropoxycarboxamido-3-{3,4-dihydro-2-(3-amino-propyl)-(2R)-2H-1,4-benzoxazin-3-on-6-yl}-propionsäure,
(2S)-2-Isobutoxycarboxamido-3-{3,4-dihydro-2-(3-amino-propyl)-(2R)-2H-1,4-benzoxazin-3-on-6-yl}-propionsäure,
(2S)-2-Neopentyloxycarboxamido-3-{3,4-dihydro-2-(3-amino-propyl)-(2R)-2H-1,4-benzoxazin-3-on-6-yl}-propionsäure, FAB 408;
(2S)-2-Benzyloxycarboxamido-3-{3,4-dihydro-2-(3-amino-propyl)-(2R)-2H-1,4-benzoxazin-3-on-6-yl}-propionsäure,
(2S)-2-Benzylsulfonamido-3-{3,4-dihydro-2-(3-amino-propyl)-(2R)-2H-1,4-benzoxazin-3-on-6-yl}-propionsäure,
(2S)-2-Benzolsulfonamido-3-{3,4-dihydro-2-(3-amino-propyl)-(2R)-2H-1,4-benzoxazin-3-on-6-yl}-propionsäure.

Analog Beispiel 3 erhält man aus den letztgenannten Propionsäuren durch Umsetzung mit DPFN die nachstehenden Verbindungen
(2S)-2-(2,3,5,6-Tetramethylphenylsulfonamido)-3-{3,4-dihydro-2-(3-guanidino-propyl)-(2R)-2H-1,4-benzoxazin-3-on-6-yl}-propionsäure,
(2S)-2-(3-Chlor-6-methoxy-phenylsulfonamido)-3-{3,4-dihydro-2-(3-guanidino-propyl)-(2R)-2H-1,4-benzoxazin-3-on-6-yl}-propionsäure,
(2S)-2-(1-Naphthylsulfonamido)-3-{3,4-dihydro-2-(3-guanidinopropyl)-(2R)-2H-1,4-benzoxazin-3-on-6-yl}-propionsäure,
(2S)-2-(2,3,5,6-Tetramethylphenylsulfo.namido)-3-{3,4-dihydro-2-(3-guanidino-propyl)-(2R)-2H-1,4-benzoxazin-3-on-6-yl}-propionsäure,
(2S)-2-[(R)-Campher-10-yl-sulfonamido)-3-{3,4-dihydro-2-(3-guanidino-propyl)-(2R)-2H-1,4-benzoxazin-3-on-6-yl}-propionsäure,
(2S)-2-Butylsulfonamido-3-{3,4-dihydro-2-(3-guanidino-propyl)-(2R)-2H-1,4-benzoxazin-3-on-6-yl}-propionsäure, FAB 456;
(2S)-2-Isopropoxycarboxamido-3-{3,4-dihydro-2-(3-guanidino-propyl)-(2R)-2H-1,4-benzoxazin-3-on-6-yl}-propionsäure,
(2S)-2-Isobutoxycarboxamido-3-{3,4-dihydro-2-(3-guanidino-propyl)-(2R)-2H-1,4-benzoxazin-3-on-6-yl}-propionsäure,
(2S)-2-Neopentyloxycarboxamido-3-{3,4-dihydro-2-(3-guanidinopropyl)-(2R)-2H-1,4-benzoxazin-3-on-6-yl}-propionsäure, FAB 450;
(2S)-2-Benzyloxycarboxamido-3-{3,4-dihydro-2-(3-guanidino-propyl)-(2R)-2H-1,4-benzoxazin-3-on-6-yl}-propionsäure,
(2S)-2-Benzylsulfonamido-3-{3,4-dihydro-2-(3-guanidino-propyl)-(2R)-2H-1,4-benzoxazin-3-on-6-yl}-propionsäure und
(2S)-2-Benzolsulfonamido-3-{3,4-dihydro-2-(3-guanidino-propyl)-(2R)-2H-1,4-benzoxazin-3-on-6-yl}-propionsäure.

Die nachfolgenden Beispiele betreffen pharmazeutische Zubereitungen:

**Beispiel A: Injektionsgläser**

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4 \cdot 2\,H_2O$, 28,48 g $Na_2HPO_4 \cdot 12\,H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

**Beispiel I: Inhalations-Spray**

Man löst 14 g Wirkstoff der Formel I in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I

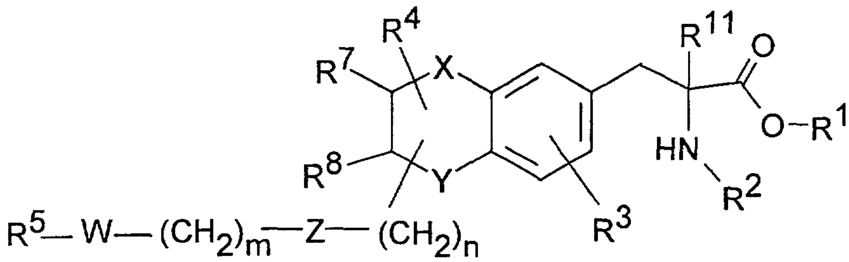

I

worin

| | |
|---|---|
| $R^1$ | H, Alkyl mit 1-6 C-Atomen oder Benzyl, |
| $R^2$ | $R^{10}$, CO-$R^{10}$, $COOR^6$, $COOR^{10}$, $SO_2R^6$ oder $SO_2R^{10}$, |
| $R^3$ | H, |
| $R^4$ | H oder =O, |
| $R^5$ | $NH_2$, $H_2N$-C(=NH) oder $H_2N$-(C=NH)-NH, wobei die primären Aminogruppen auch mit konventionellen Aminoschutzgruppen versehen sein können, oder ein-, zwei- oder dreifach durch $R^{10}$, CO-$R^{10}$, $COOR^{10}$ oder $SO_2R^{10}$ substituiert sein können, oder $R^6$, |
| $R^7$, $R^8$ | jeweils unabhängig voneinander fehlt oder H, |
| $R^7$ und $R^8$ | zusammen auch eine Bindung, |
| X | O oder N, |
| Y | O, |
| W, Z | jeweils unabhängig voneinander fehlt, O, S, $NR^1$, C(=O), CONH, NHCO, C(=S)NH, NHC(=S), C(=S), $SO_2NH$, $NHSO_2$ oder CA=CA', |
| $R^6$ | 1H-Imidazol-2-yl, Thiazol-2-yl, 1H-Benzimidazol-2-yl, 2H-Pyrazol-2-yl, 1H-Tetrazol-5-yl, 2-Imino-imidazolidin-4-on-5-yl, 1-Alkyl-1,5-dihydro-imidazol-4-on-2-yl, Pyrimidin-2-yl oder 1,4,5,6-Tetrahydropyrimidin-2-yl, |
| $R^9$ | H, Hal, OA, NHA, NAA', NHAcyl, OAcyl, CN, $NO_2$, SA, SOA, $SO_2A$, $SO_2Ar$ oder $SO_3H$, |
| $R^{10}$ | H, A, Ar oder Aralkylen mit 7-14 C-Atomen, |
| $R^{11}$ | H oder Alkyl mit 1-6 C-Atomen, |
| A, A' | jeweils unabhängig voneinander H oder unsubstituiertes oder ein-, zwei- oder dreifach durch $R^9$ substituiertes Alkyl oder Cycloalkyl mit bis zu 15 C-Atomen und worin eine, zwei- oder drei Methylengruppen durch N, O und/oder S ersetzt sein können, |
| Ar | unsubstituiertes oder ein-, zwei- oder dreifach durch A und/oder $R^9$ substituiertes ein- oder zweikerniges aromatisches Ringsystem mit 0, 1, 2, 3 oder 4 N-, O- und/oder S-Atomen, |
| Acyl | Formyl, Acetyl, Propionyl, Butyryl, Trifluoracetyl oder Benzoyl, |
| Hal | F, Cl, Br oder I und |

m, n jeweils unabhängig voneinander 0, 1, 2, 3 oder 4

bedeuten,
sowie deren physiologisch unbedenklichen Salze.

**2.** Enantiomere oder Diastereomere der Verbindungen der Formel I gemäß Anspruch 1.

**3.** Verbindungen der Formel I gemäß Anspruch 1

a) (2S)-2-Benzyloxycarboxamido-3-(2-guanidinomethyl-1,4-benzodioxan-6-yl)-propionsäure;

b) (2S)-2-tert.-Butyloxycarboxamido-3-[3,4-dihydro-2-(2-guanidino-2-oxoethyl)-2H-1,4-benzoxazin-3-on-6-yl]-propionsäure;

c) (2S)-2-Benzyloxycarboxamido-3-(2-guanidino-acetamidomethyl-1,4-benzodioxan-6-yl)-propionsäure;

d) (2S)-2-tert.-Butyloxycarboxamido-3-{3,4-dihydro-2-[N-(2-imidazolyl)-carbamoylmethyl]-2H-1,4-benzoxazin-3-on-6-yl}propionsäure;

e) (2S)-2-tert.-Butyloxycarboxamido-3-{3,4-dihydro-2-[N-(2-benzimidazolyl)-carbamoylmethyl]-2H-1,4-benzoxazin-3-on-6-yl}propionsäure;

f) (2S)-2-tert.-Butyloxycarboxamido-3-{3,4-dihydro-2-[2-(2-imino-4-oxo-imidazolidin-5-yl)-ethyl]-2H-1,4-benzoxazin-3-on-6-yl}propionsäure;

g) (2S)-2-tert.-Butyloxycarboxamido-3-{3,4-dihydro-2-[N-(2-imidazolyl)-carbamoylethyl]-(2S)-2H-1,4-benzoxazin-3-on-6-yl}propionsäure;

h) (2S)-2-[(R)-Campher-sulfonamido]-3-{3,4-dihydro-2-[N-(2-benzimidazolyl)-carbamoylmethyl]-2H-1,4-benzoxazin-3-on-6-yl}propionsäure;

sowie deren physiologisch unbedenklichen Salze.

**4.** Verfahren zur Herstellung von Verbindungen der Formel I nach Anspruch 1 sowie ihrer Salze, **dadurch gekennzeichnet**,

a) daß man eine Verbindung der Formel 1 aus einem ihrer funktionellen Derivate durch Behandeln mit einem solvolysierenden oder hydrogenolysierenden Mittel in Freiheit setzt,
oder

b) daß man eine Verbindung der Formel II

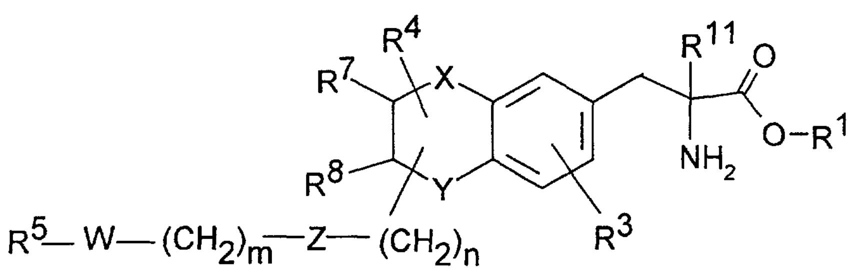

II

worin $R^1$, $R^3$, $R^4$, $R^5$, $R^7$, $R^8$, $R^{11}$, W, X, Y, Z, m und n die in Anspruch 1 angegebenen Bedeutungen haben, mit einer Verbindung der Formel III

$R^2$-L III

worin
$R^2$ die in Anspruch 1 angegebene Bedeutung hat
und L Cl, Br, I, OH oder eine reaktionsfähig veresterte OH-Gruppe bedeutet,
umsetzt,
oder

c) daß man einen Ester der Formel I verseift,
oder

d) daß man einen Rest $R^1$ und/oder $R^5$ in einen anderen Rest $R^1$ und/oder $R^5$ umwandelt,
und/oder

e) daß man eine basische oder saure Verbindung der Formel I durch Behandeln mit einer Säure oder Base in eines ihrere Salze überführt.

**5.** Verfahren zur Herstellung einer pharmazeutischen Zubereitung, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

**6.** Pharmazeutische Zubereitung, **gekennzeichnet durch** einen Gehalt an mindestens einer Verbindung der Formel I nach Anspruch 1 und/oder einem ihrer physiologisch unbedenklichen Salze.

**7.** Verbindungen der Formel I nach Anspruch 1 und ihre physiologisch unbedenklichen Salze als GPIIb/IIIa-Antagonisten zur Bekämpfung von Thrombosen, Herzinfarkt, koronaren Herzerkrankungen und Arteriosklerose.

**8.** Verbindungen der Formel I nach Anspruch 1 und ihre physiologisch unbedenklichen Salze als $\alpha_V$-Integrininhibitoren zur Bekämpfung von pathologisch angiogenen Erkrankungen, Thrombosen, Herzinfarkt, koronaren Herzerkrankungen, Arteriosklerose, Tumoren, Osteoporose, Entzündungen und Infektionen.

**9.** Verbindungen der Formel I nach Anspruch 1 und ihre physiologisch unbedenklichen Salze, worin $R^2$ die Bedeutung Campher-10-yl hat, als $\alpha_V$-Integrininhibitoren zur Bekämpfung von pathologisch angiogenen Erkrankungen, Thrombosen, Herzinfarkt, koronaren Herzerkrankungen, Arteriosklerose, Tumoren, Osteoporose, Entzündungen und Infektionen.

**10.** Verwendung von Verbindungen der Formel I nach Anspruch 1 und/oder ihre physiologisch unbedenklichen Salze zur Herstellung eines Arzneimittels.

**11.** Verbindungen der Formel I nach Anspruch 1 und/oder ihrer physiologisch unbedenklichen Salze zur Herstellung eines Arzneimittels zur Verwendung als $\alpha_V$-Integrin-Inhibitor.

**Claims**

**1.** Compounds of the formula I

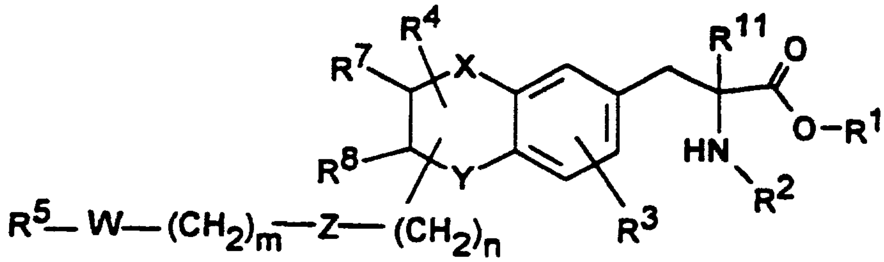

I

wherein

| | |
|---|---|
| $R^1$ | is H, alkyl having 1-6 C atoms or benzyl, |
| $R^2$ | is $R^{10}$, CO-$R^{10}$, $COOR^6$, $COOR^{10}$, $SO_2R^6$ or $SO_2R^{10}$, |
| $R^3$ | is H, |
| $R^4$ | is H or =O, |
| $R^5$ | is $NH^2$, $H_2N$-C(=NH) or $H_2N$-(C=NH)-NH, where the primary amino groups can also be provided with conventional amino protective groups or can be mono-, di- or trisubstituted by $R^{10}$, CO-$R^{10}$, $COOR^{10}$ or $SO_2R^{10}$,<br>or $R^6$, |
| $R^7$, $R^8$ | are each independently of one another absent or H, |
| $R^7$ and $R^8$ | together are also a bond, |
| X | is O or N, |
| Y | is O, |
| W, Z | are each independently of one another absent, O, S, $NR^1$, C(=O), CONH, NHCO, C(=S)NH, NHC(=S), C(=S), $SO_2NH$, $NHSO_2$ or CA=CA', |
| $R^6$ | is 1H-imidazol-2-yl, thiazol-2-yl, 1H-benzimidazol-2-yl, 2H-pyrazol-2-yl, 1H-tetrazol-5-yl, 2-iminoimidazolidin-4-on-5-yl, 1-alkyl-1, 5-dihydroimidazol-4-on-2-yl, pyrimidin-2-yl or 1, 4, 5, 6-tetrahydropyrimidin-2-yl, |
| $R^9$ | is H, Hal, OA, NHA, NAA', NHacyl, Oacyl, CN, $NO_2$, SA, SOA, $SO_2A$, $SO_2Ar$ or $SO_3H$, |
| $R^{10}$ | is H, A, Ar or aralkylene [sic] having 7-14 C atoms, |
| $R^{11}$ | is H or alkyl having 1-6 C atoms, |
| A, A' | are each independently of one another H or unsubstituted or mono-, di- or tri-$R^9$- substituted alkyl or cycloalkyl, each of which has up to 15 C atoms and in which one, two or three methylene groups can be replaced by N, O and/or S, |
| Ar | is unsubstituted or mono-, di- or tri-A- and/or $R^9$-substituted mono- or binuclear aromatic ring system having 0, 1, 2, 3 or 4 N, O and/or S atoms, |
| Acyl | is formyl, acetyl, propionyl, butyryl, trifluoroacetyl or benzoyl, |
| Hal | is F, Cl, Br or I and |

m, n are each independently of one another 0, 1, 2, 3 or 4,

and the physiologically acceptable salts thereof.

**2.** Enantiomers or diastereomers of the compounds of the formula I according to Claim 1.

**3.** Compounds of the formula I according to Claim 1

a) (2S)-2-benzyloxycarboxamido-3-(2-guanidinomethyl-1,4-benzodioxan-6-yl)propionic acid;

b) (2S)-2-tert-butyloxycarboxamido-3-[3,4-dihydro-2-(2-guanidino-2-oxoethyl)-2H-1,4-benzoxazin-3-on-6-yl] propionic acid;

c) (2S)-2-benzyloxycarboxamido-3-(2-guanidinoacetamidomethyl-1,4-benzodioxan-6-yl)propionic acid;

d) (2S)-2-tert-butyloxycarboxamido-3-{3,4-dihydro-2-[N-(2-imidazolyl)carbamoylmethyl]-2H-1,4-benzoxazin-3-on-6-yl)propionic acid;

e) (2S)-2-tert-butyloxycarboxamido-3-{3,4-dihydro-2-[N-(2-benzimidazolyl)carbamoylmethyl]-2H-1,4-benzoxazin-3-on-6-yl)propionic acid;

f) (2S)-2-tert-butyloxycarboxamido-3-{3,4-dihydro-2-[2-(2-imino-4-oxoimidazolidin-5-yl)ethyl]-2H-1,4-benzoxazin-3-on-6-yl}propionic acid;

g) (2S)-2-tert-butyloxycarboxamido-3-{3,4-dihydro-2-[N-(2-imidazolyl)carbamoylethyl]-(2S)-2H-1, 4-benzoxazin-3-on-6-yl}propionic acid;

h) (2S)-2-[(R)-camphorsulfonamido]-3-{3,4-dihydro-2-[N-(2-benzimidazolyl)carbamoylmethyl]-2H-1,4-benzoxazin-3-on-6-yl}propionic acid;

and their physiologically acceptable salts.

**4.** Process for the preparation of compounds of the formula I according to Claim 1 and of their salts, **characterized in that**

a) **in that** a compound of the formula I is liberated from one of its functional derivatives by treatment with a solvolysing or hydrogenolysing agent,

or

b) **in that** a compound of the formula II

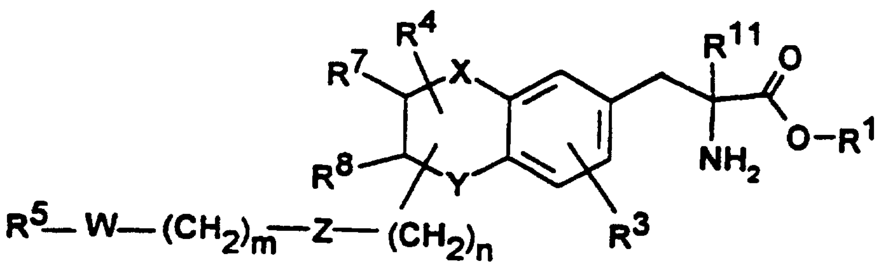

II

in which $R^1$, $R^3$, $R^4$, $R^5$, $R^7$, $R^8$, $R^{11}$, W, X, Y, Z, m and n have the meanings stated in Claim 1, is reacted with a compound of the formula III

$R^2$-L III

in which
$R^2$ has the meaning stated in Claim 1,
and L is Cl, Br, I, OH or a reactively esterified OH group,
or

c) **in that** an ester of the formula I is hydrolysed,
or

d) **in that** a radical $R^1$ and/or $R^5$ is converted into another radical $R^1$ and/or $R^5$,
and/or

e) **in that** a basic or acidic compound of the formula I is converted by treatment with an acid or base into one of the salts thereof.

**5.** Process for the production of a pharmaceutical composition, **characterized in that** a compound of the formula I according to Claim 1 and/or one of its physiologically acceptable salts is converted together with at least one solid, liquid or semiliquid excipient or ancillary substance into a suitable dosage form.

**6.** Pharmaceutical composition **characterized by** a content of at least one compound of the formula I according to Claim 1 and/or one of its physiologically acceptable salts.

**7.** Compounds of the formula I according to Claim 1 and their physiologically acceptable salts as GPIIb/IIIa antagonists for controlling thromboses, myocadial infarct, coronary heart disease and arteriosclerosis.

**8.** Compounds of the formula I according to Claim 1 and their physiologically acceptable salts as $\alpha_v$ integrin inhibitors for controlling pathologically angiogenic disorders, thromboses, myocardial infarct, coronary heart disease, arteriosclerosis, tumours, osteoporosis, inflammations and infections.

**9.** Compounds of the formula I according to Claim 1 and their physiologically acceptable salts, in which $R^2$ means camphor-10-yl, as $\alpha_v$ integrin inhibitors for controlling pathologically angiogenic disorders, thromboses, myocardial infarct, coronary heart disease, arteriosclerosis, tumours, osteoporosis, inflammations and infections.

**10.** Use of compounds of the formula I according to Claim 1 and/or their physiologically acceptable salts for producing a pharmaceutical.

**11.** Compounds of the formula I according to Claim 1 and/or their physiologically acceptable salts for producing a pharmaceutical for use as $\alpha_v$ integrin inhibitor.

## Revendications

**1.** Composés de formule I

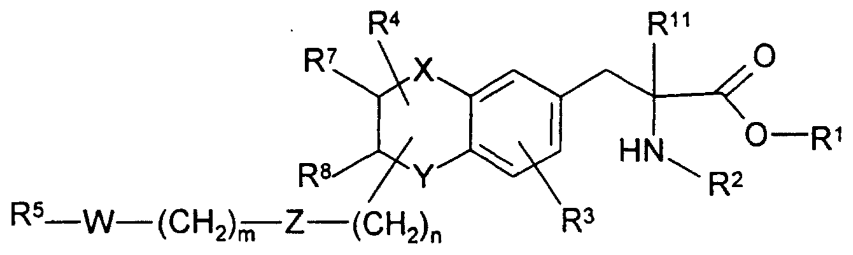

I

où

- $R^1$ représente H, un alkyle comportant 1 à 6 atomes de C ou le benzyle,
- $R^2$ représente $R^{10}$, CO-$R^{10}$, $COOR^6$, $COOR^{10}$, $SO_2R^6$ ou $SO_2R^{10}$,
- $R^3$ représente H,
- $R^4$ représente H ou =O,
- $R^5$ représente $NH_2$, $H_2N$-C(=NH) ou $H_2N$-(C=NH)-NH, les groupes amino primaires pouvant être également munis de groupes protecteurs de l'amino classiques ou pouvant être mono-, di- ou trisubstitués par $R^{10}$, CO-$R^{10}$, $COOR^{10}$ ou $SO_2R^{10}$ ou représente $R^6$,
- $R^7$, $R^8$ indépendamment l'un de l'autre, manquent ou représentent H,
- $R^7$ et $R^8$ représentent ensemble également une liaison,
- X représente O ou N,
- Y représente O,
- W, Z indépendamment l'un de l'autre, manquent ou représentent O, S, $NR^1$, C(=O), CONH, NHCO, C(=S)NH, NHC(=S), C(=S), $SO_2NH$, $NHSO_2$ ou CA=CA',
- $R^6$ représente le 1H-imidazol-2-yle, le thiazol-2-yle, le 1H-benzimidazol-2-yle, le 2H-pyrazol-2-yle, le 1H-tétrazol-5-yle, le 2-iminoimidazolidin-4-on-5-yle, le 1-alkyl-1,5-dihydroimidazol-4-on-2-yle, le pyrimidin-2-yle ou le 1,4,5,6-tétrahydropyrimidin-2-yle,
- $R^9$ représente H, Hal, OA, NHA, NAA', NHacyle, Oacyle, CN, $NO_2$, SA, SOA, $SO_2A$, $SO_2Ar$ ou $SO_3H$,
- $R^{10}$ représente H, A, Ar ou un aralkylène comportant 7 à 14 atomes de C,
- $R^{11}$ représente H ou un alkyle comportant 1 à 6 atomes de C,
- A, A' représentent, indépendamment l'un de l'autre, H ou un alkyle ou cycloalkyle comportant jusqu'à 15 atomes de C, non substitué ou mono-, di- ou trisubstitué par $R^9$ et où un, deux ou trois groupes méthylènes peuvent être remplacés par N, O et/ou S,
- Ar représente un cycle aromatique mono- ou bicyclique comportant 0, 1, 2, 3 ou 4 atomes de N, O et/ou S, non substitué ou mono-, di- ou trisubstitué par A et/ou $R^9$,
- Acyle représente le formyle, l'acétyle, le propionyle, le butyryle, le trifluoroacétyle ou le benzoyle,
- Hal représente F, Cl, Br ou I et
- m, n sont égaux, indépendamment l'un de l'autre, à 0, 1, 2, 3 ou 4,

ainsi que leurs sels physiologiquement acceptables.

**2.** Les énantiomères ou diastéréoisomères des composés de formule I selon la revendication 1.

**3.** Les composés de formule I selon la revendication 1

a) l'acide (2S)-2-benzyloxycarboxamido-3-(2-guanidinométhyt-1,4-benzodioxan-6-yl)propionique,
b) l'acide (2S)-2-tert-butyloxycarboxamido-3-[3,4-dihydro-2-(2-guanidino-2-oxoéthyl)-2H-1,4-benzoxazin-3-on-6-yl]propionique,
c) l'acide (2S)-2-benzyloxycarboxamido-3-(2-guanidinoacétamidométhyl-1,4-benzodioxan-6-yl)propionique,
d) l'acide (2S)-2-tert-butyloxycarboxamido-3-{3,4-dihydro-2-[N-(2-imidazolyl)carbamoylméthyl]-2H-1,4-benzoxazin-3-on-6-yl}propionique,
e) l'acide (2S)-2-tert-butyloxycarboxamido-3-{3,4-dihydro-2-[N-(2-benzimidazolyl)carbamoylméthyl]-2H-1,4-benzoxazin-3-on-6-yl}propionique,
f) l'acide (2S)-2-tert-butyloxycarboxamido-3-{3,4-dihydro-2-[2-(2-imino-4-oxoimidazolidin-5-yl)éthyl]-2H-1,4-benzoxazin-3-on-6-yl}propionique,
g) l'acide (2S)-2-tert-butyloxycarboxamido-3-{3,4-dihydro-2-[N-(2-imidazolyl)carbamoyléthyl]-(2S)-2H-1,4-benzoxazin-3-on-6-yl}propionique,
h) l'acide (2S)-2-[(R)-camphosulfonamido]-3-{3,4-dihydro-2-[N-(2-benzimidazolyl)carbamoylméthyl]-2H-1,4-benzoxazin-3-on-6-yl}propionique,

ainsi que leurs sels physiologiquement acceptables.

**4.** Procédé pour la préparation des composés de formule I selon la revendication 1, ainsi que de leurs sels, caractérisé

a) en ce que l'on libère un composé de formule I de l'un de ses dérivés fonctionnels par traitement avec un agent solvolysant ou hydrogénolysant,
ou

b) en ce que l'on fait réagir un composé de formule II

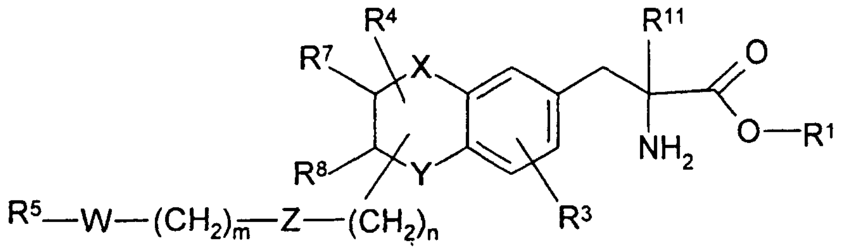

II

où
$R^1$, $R^3$, $R^4$, $R^5$, $R^7$, $R^8$, $R^{11}$, W, X, Y, Z, m et n ont les significations indiquées dans la revendication 1, avec un composé de formule III

$$R^2\text{-L} \qquad \text{III}$$

où
$R^2$ a la signification indiquée dans la revendication 1 et L représente Cl, Br, I, OH estérifié réactif
ou
c) en ce que l'on saponifie un ester de formule I,
ou
d) en ce que l'on transforme un reste $R^1$ et/ou $R^5$ en un autre reste $R^1$ et/ou $R^5$,
et/ou
e) en ce que l'on transforme un composé basique ou acide de formule I en l'un de ses sels par traitement avec un acide ou une base.

**5.** Procédé pour la fabrication d'une préparation pharmaceutique, **caractérisé en ce que** l'on met sous une forme de dosage appropriée un composé de formule I selon la revendication 1, et/ou l'un de ses sels physiologiquement acceptables, associé à au moins un support ou un adjuvant solide, liquide ou semi-liquide.

**6.** Préparation pharmaceutique **caractérisée en ce qu'**elle contient au moins un composé de formule I selon la revendication 1 et/ou l'un de ses sels physiologiquement acceptables.

**7.** Composés de formule I selon la revendication 1 et leurs sels physiologiquement acceptables comme antagonistes des GPIIb/IIIa pour lutter contre les thromboses, l'infarctus du myocarde, les maladies coronaires et l'artériosclérose.

**8.** Composés de formule I selon la revendication 1 et leurs sels physiologiquement acceptables comme inhibiteurs de l'$\alpha_v$-intégrine pour lutter contre les maladies angiogènes pathologiques, les thromboses, l'infarctus du myocarde, les maladies coronaires, l'artériosclérose, les tumeurs, l'ostéoporose, les inflammations et les infections.

**9.** Composés de formule I selon la revendication 1 et leurs sels physiologiquement acceptables où $R^2$ représente le camphor-10-yle, comme inhibiteurs de l'$\alpha_v$-intégrine, pour lutter contre les maladies angiogènes pathologiques, les thromboses, l'infarctus du myocarde, les maladies coronaires, l'artériosclérose, les tumeurs, l'ostéoporose, les inflammations et les infections.

**10.** Utilisation des composés de formule I selon la revendication 1 et/ou de leurs sels physiologiquement acceptables pour la fabrication d'un médicament.

**11.** Composés de formule I selon la revendication 1 et/ou leurs sels physiologiquement acceptables pour la fabrication d'un médicament destiné à être utilisé comme inhibiteur de l'$\alpha_v$-intégrine.